# EUROPEAN PATENT APPLICATION

(11) **EP 1 723 970 A1**
(43) Date of publication of application: **22.11.2006**
(21) Application number: 05720370.5
(22) Date of filing: 09.03.2005
(51) Int. Cl.: A61K 45/00, A61K 31/7088, A61K 31/7105, A61K 39/395, A61K 48/00, A61P 35/00, A61P 35/04, A61P 43/00

(54) **MEDICINAL COMPOSITION CONTAINING CXCR3 INHIBITOR**

(30) Priority: 09.03.2004 JP 2004065612
(71) Applicant: Kyoto University, Kyoto-shi, Kyoto 606-8501 (JP)
(72) Inventor: TAKETO, Makoto, Graduate School of Medicine, Kyoto-shi, Kyoto 6068501 (JP); KAWADA, Kenji, Graduate School of Medicine, Kyoto-shi, Kyoto 6068501 (JP)
(74) Representative: Helbing, Jörg
(86) International application number: PCT/JP2005/004098
(87) International publication number: WO 2005/084708

(57) **Abstract**

A pharmaceutical composition for the treatment of cancer of which survival and/or metastasis is under the influence of signal transduction mediated by a chemokine receptor CXCR3, which comprises an agent to inhibit CXCR3, and a method of treatment and diagnosis of such cancer.

SEQUENCE LISTING

<110>  Kyoto University

<120>  Pharmaceutical composition comprising a CXCR3 inhibitor

<130>  665078

<150> JP JP 2004-065612
<151> 2004-03-09

<160>  34

<170>  PatentIn version 3.1

<210>  1
<211>  368
<212>  PRT
<213>  Homo sapiens

<400>  1

Met Val Leu Glu Val Ser Asp His Gln Val Leu Asn Asp Ala Glu Val
1               5                   10                  15

Ala Ala Leu Leu Glu Asn Phe Ser Ser Ser Tyr Asp Tyr Gly Glu Asn
            20                  25                  30

Glu Ser Asp Ser Cys Cys Thr Ser Pro Pro Cys Pro Gln Asp Phe Ser
        35                  40                  45

Leu Asn Phe Asp Arg Ala Phe Leu Pro Ala Leu Tyr Ser Leu Leu Phe
    50                  55                  60

Leu Leu Gly Leu Leu Gly Asn Gly Ala Val Ala Ala Val Leu Leu Ser
65                  70                  75                  80

Arg Arg Thr Ala Leu Ser Ser Thr Asp Thr Phe Leu Leu His Leu Ala
                85                  90                  95

Val Ala Asp Thr Leu Leu Val Leu Thr Leu Pro Leu Trp Ala Val Asp
            100                 105                 110

Ala Ala Val Gln Trp Val Phe Gly Ser Gly Leu Cys Lys Val Ala Gly
        115                 120                 125

Ala Leu Phe Asn Ile Asn Phe Tyr Ala Gly Ala Leu Leu Leu Ala Cys
    130                 135                 140

Ile Ser Phe Asp Arg Tyr Leu Asn Ile Val His Ala Thr Gln Leu Tyr
145                 150                 155                 160

Arg Arg Gly Pro Pro Ala Arg Val Thr Leu Thr Cys Leu Ala Val Trp
                165                 170                 175

Gly Leu Cys Leu Leu Phe Ala Leu Pro Asp Phe Ile Phe Leu Ser Ala
            180                 185                 190

His His Asp Glu Arg Leu Asn Ala Thr His Cys Gln Tyr Asn Phe Pro
        195                 200                 205

Gln Val Gly Arg Thr Ala Leu Arg Val Leu Gln Leu Val Ala Gly Phe
    210                 215                 220

Leu Leu Pro Leu Leu Val Met Ala Tyr Cys Tyr Ala His Ile Leu Ala
225                 230                 235                 240

Val Leu Leu Val Ser Arg Gly Gln Arg Arg Leu Arg Ala Met Arg Leu
                245                 250                 255

Val Val Val Val Val Val Ala Phe Ala Leu Cys Trp Thr Pro Tyr His
            260                 265                 270

Leu Val Val Leu Val Asp Ile Leu Met Asp Leu Gly Ala Leu Ala Arg
        275                 280                 285

Asn Cys Gly Arg Glu Ser Arg Val Asp Val Ala Lys Ser Val Thr Ser
    290                 295                 300

Gly Leu Gly Tyr Met His Cys Cys Leu Asn Pro Leu Leu Tyr Ala Phe
305                 310                 315                 320

Val Gly Val Lys Phe Arg Glu Arg Met Trp Met Leu Leu Leu Arg Leu
                325                 330                 335

Gly Cys Pro Asn Gln Arg Gly Leu Gln Arg Gln Pro Ser Ser Ser Arg
            340                 345                 350

Arg Asp Ser Ser Trp Ser Glu Thr Ser Glu Ala Ser Tyr Ser Gly Leu
        355                 360                 365

<210>  2
<211>  1670
<212>  DNA
<213>  Homo sapiens

<400>  2
ccaaccacaa gcaccaaagc agaggggcag gcagcacacc acccagcagc cagagcacca      60

gcccagccat ggtccttgag gtgagtgacc accaagtgct aaatgacgcc gaggttgccg     120

ccctcctgga gaacttcagc tcttcctatg actatggaga aaacgagagt gactcgtgct     180

gtacctcccc gccctgccca caggacttca gcctgaactt cgaccgggcc ttcctgccag     240

ccctctacag cctcctcttt ctgctggggc tgctgggcaa cggcgcggtg gcagccgtgc     300

tgctgagccg gcggacagcc ctgagcagca ccgacacctt cctgctccac ctagctgtag     360

cagacacgct gctggtgctg acactgccgc tctgggcagt ggacgctgcc gtccagtggg     420

tctttggctc tggcctctgc aaagtggcag gtgccctctt caacatcaac ttctacgcag     480

gagccctcct gctggcctgc atcagctttg accgctacct gaacatagtt catgccaccc     540

agctctaccg ccgggggccc ccggcccgcg tgaccctcac ctgcctggct gtctgggggc     600

tctgcctgct tttcgccctc ccagacttca tcttcctgtc ggcccaccac gacgagcgcc     660

tcaacgccac ccactgccaa tacaacttcc cacaggtggg ccgcacggct ctgcgggtgc     720

tgcagctggt ggctggcttt ctgctgcccc tgctggtcat ggcctactgc tatgcccaca     780

tcctggccgt gctgctggtt tccaggggcc agcggcgcct gcgggccatg cggctggtgg     840

tggtggtcgt ggtggccttt gccctctgct ggacccccta tcacctggtg gtgctggtgg     900

acatcctcat ggacctgggc gctttggccc gcaactgtgg ccgagaaagc agggtagacg     960

tggccaagtc ggtcacctca ggcctgggct acatgcactg ctgcctcaac ccgctgctct    1020

atgcctttgt aggggtcaag ttccgggagc ggatgtggat gctgctcttg cgcctgggct    1080

gccccaacca gagagggctc cagaggcagc catcgtcttc ccgccgggat tcatcctggt    1140

ctgagacctc agaggcctcc tactcgggct tgtgaggccg gaatccgggc tcccctttcg    1200

cccacagtct gacttccccg cattccaggc tcctccctcc ctctgccggc tctggctctc    1260

cccaatatcc tcgctcccgg gactcactgg cagccccagc accaccaggt ctcccgggaa    1320

gccaccctcc cagctctgag gactgcacca ttgctgctcc ttagctgcca agccccatcc    1380

tgccgcccga ggtggctgcc tggagcccca ctgcccttct catttggaaa ctaaaacttc    1440

atcttcccca agtgcgggga gtacaaggca tggcgtagag ggtgctgccc catgaagcca   1500

cagcccaggc ctccagctca gcagtgactg tggccatggt ccccaagacc tctatatttg   1560

ctcttttatt tttatgtcta aaatcctgct taaaactttt caataaacaa gatcgtcagg   1620

accaaaaaaa aaaaaaaaaa aaaaaaaaaa aaaaaaaaaa aaaaaaaaaa              1670

<210>  3
<211>  22
<212>  DNA
<213>  Artificial sequence

<220>
<223>  DNA primer for PCR

<400>  3
gccggagcac cagccaagcc at                                                22

<210>  4
<211>  20
<212>  DNA
<213>  Artificial sequence

<220>
<223>  DNA primer for PCR

<400>  4
aggtggagca ggaaggtgtc                                                   20

<210>  5
<211>  21
<212>  DNA
<213>  Artificial sequence

<220>
<223>  DNA primer for PCR

<400>  5
ctggaatcta ggaagtacca c                                                 21

<210>  6
<211>  21
<212>  DNA
<213>  Artificial sequence

<220>
<223>  DNA primer for PCR

<400>  6
ccaaaaaggc ataaagcacc g                                                 21

<210>  7
<211>  21
<212>  DNA
<213>  Artificial sequence

<220>
<223>  DNA primer for PCR

<400>  7
aagccatgta ccttgaggtt a                                                21


<210>  8
<211>  22
<212>  DNA
<213>  Artificial sequence

<220>
<223>  DNA primer for PCR

<400>  8
cagacagaga ccccatacaa gc                                               22


<210>  9
<211>  24
<212>  DNA
<213>  Artificial sequence

<220>
<223>  DNA primer for PCR

<400>  9
cctagctcag ttctctggac atga                                             24


<210>  10
<211>  21
<212>  DNA
<213>  Artificial sequence

<220>
<223>  DNA primer for PCR

<400>  10
tcgcaggcct ctaagatacg a                                                21


<210>  11
<211>  28
<212>  DNA
<213>  Artificial sequence

<220>
<223>  DNA primer for PCR

<400>  11
ctgcctgggc cacagttcac ctctaatt                                          28


<210>  12
<211>  23
<212>  DNA
<213>  Artificial sequence

<220>
<223>  DNA primer for PCR

<400>  12
agaactcagc tctgccatga agt                                               23


<210>  13
<211>  21
<212>  DNA
<213>  Artificial sequence

<220>
<223>  DNA primer for PCR

<400>  13
aactccacac tgctccagga a                                                 21


<210>  14
<211>  26
<212>  DNA
<213>  Artificial sequence

<220>
<223>  DNA primer for PCR

<400>  14
cgctgttctt ttccttttgg gcatca                                            26


<210>  15
<211>  23
<212>  DNA
<213>  Artificial sequence

<220>
<223>  DNA primer for PCR

<400>  15
ccagtgagaa tgagggccat agg                                               23


<210>  16
<211>  19
<212>  DNA

<213>  Artificial sequence

<220>
<223>  DNA primer for PCR

<400>  16
ctcaacacgt gggcaggat                                                    19


<210>  17
<211>  25
<212>  DNA
<213>  Artificial sequence

<220>
<223>  DNA primer for PCR

<400>  17
aagcttgaaa tcatccctgc gagcc                                             25


<210>  18
<211>  21
<212>  DNA
<213>  Artificial sequence

<220>
<223>  DNA primer for PCR

<400>  18
caggaaggtc acagccatag c                                                 21


<210>  19
<211>  19
<212>  DNA
<213>  Artificial sequence

<220>
<223>  DNA primer for PCR

<400>  19
caaagacagc gcccctgtt                                                    19


<210>  20
<211>  29
<212>  DNA
<213>  Artificial sequence

<220>
<223>  DNA primer for PCR

<400>  20
ccacagctgc tcaaggcttc cttatgttc                                         29

<210>  21
<211>  20
<212>  DNA
<213>  Artificial sequence

<220>
<223>  DNA primer for PCR

<400>  21
caaagcagcc acctcatgct                                                   20


<210>  22
<211>  20
<212>  DNA
<213>  Artificial sequence

<220>
<223>  DNA primer for PCR

<400>  22
atggccgtgc agatgtaatg                                                   20


<210>  23
<211>  23
<212>  DNA
<213>  Artificial sequence

<220>
<223>  DNA primer for PCR

<400>  23
tccacaccct tgccctgctt caa                                               23


<210>  24
<211>  27
<212>  DNA
<213>  Artificial sequence

<220>
<223>  DNA primer for PCR

<400>  24
tgaattcaagc catgtacctt gaggtta                                           27


<210>  25
<211>  27
<212>  DNA
<213>  Artificial sequence

<220>
<223>  DNA primer for PCR

<400>  25
ctcgagaatt acaagcccag gtaggag                                          27


<210>  26
<211>  25
<212>  DNA
<213>  Artificial sequence

<220>
<223>  DNA primer for PCR

<400>  26
cactgactct ctctgcctat tggtc                                            25


<210>  27
<211>  21
<212>  DNA
<213>  Artificial sequence

<220>
<223>  DNA primer for PCR

<400>  27
aggagtggac agatccccaa a                                                21


<210>  28
<211>  30
<212>  DNA
<213>  Artificial sequence

<220>
<223>  DNA probe for PCR

<400>  28
ctacccttgg acccagaggt tctttgagtc                                        21


<210>  29
<211>  21
<212>  DNA
<213>  Homo sapiens

<400>  29
aagtggcagg tgccctcttc a                                                21


<210>  30
<211>  21
<212>  RNA
<213>  Artificial sequence

<220>

<223>  siRNA

<400>  30
guggcaggug cccucuucat t                                                21


<210>  31
<211>  21
<212>  RNA
<213>  Artificial sequence

<220>
<223>  siRNA

<400>  31
ugaagagggc accugccact t                                                21


<210>  32
<211>  21
<212>  DNA
<213>  Homo sapiens

<400>  32
aacgagagtg actcgtgctg t                                                21


<210>  33
<211>  21
<212>  RNA
<213>  Artificial sequence

<220>
<223>  siRNA

<400>  33
cgagagugac ucgugcugut t                                                21


<210>  34
<211>  21
<212>  RNA
<213>  Artificial sequence

<220>
<223>  siRNA

<400>  34
acagcacgag ucacucucgt t                                                21

## Description

### TECHNICAL FIELD

The present invention relates to prevention and treatment of cancer, and specifically to a pharmaceutical composition for the treatment of cancer of which survival and/or metastasis is under the influence of signal transduction mediated by a chemokine receptor CXCR3, and a method of treatment and diagnosis of such cancer.

### BACKGROUND ART

Lymph node metastasis is one of the earliest features of tumor cell dissemination in most human cancers, and lymph node metastasis is found in 60% or more cases of malignant melanoma. Metastasis of cancer is largely classified into lymphogenous and hematogenous metastases, and they occur rather simultaneously than independently. Lymphaticovenous shunts or the like often bypass regional (local) lymph nodes, thereby allowing dissemination of malignant cells into systemic circulation at an early stage. The biological aggressiveness of cancer (malignant tumor) can be evaluated on the basis of the expression site, rate of progress, and metastatic capacity. Thus, metastasis of cancer cells to the regional lymph node appears to be a reflection of the biological aggressiveness of cancers, and its assessment is critical for predicting prognosis and setting up therapeutic strategies.

Malignant tumors are generally metastatic and the currently available surgical and medical therapy for metastatic cancer are not effective enough. Actually, it is said that most of available chemotherapies effective on primary cancer have low or no effects on recurrent metastatic cancer. Further, the earlier the treatment begins, the higher the successful rate, and hence early detection and treatment are significant for the effective treatment of metastatic cancer.

Diagnosis of cancer is performed by known methods using tumor markers, the positron computed tomography, and the like; however these methods cannot necessarily detect cancer certainly. In particular, many metastatic cancers are refractory, and the success rate of treatment thereof is generally low compared to that in the treatment of primary cancer. Further, detection of cancer tends to be delayed in the case of distant metastasis through lymph nodes. Thus, for the development of cancer therapy, it is inevitable to establish an effective method for the prevention of metastasis, especially for the prevention of lymph node metastasis and/or treatment of metastatic cancer, as well as the treatment of detected cancer.

It is known that chemokine-receptor-mediated signal transduction is deeply involved in the expression of function of cancer cells. Chemokines constitute a family of structurally related small cytokines having molecular weight of about 10,000 and divided into four families, CXC, CC, C and CX3C, based on the positions of four conserved-cysteine residues. Chemokines act through seven transmembrane spanning G protein-coupled receptors. Examples of disorders for which chemokines and their receptors are responsible include inflammation, infection, tissue injury, allergy, and cardiovascular diseases and malignant tumors. The role of chemokines in malignant tumors appears complex. Whereas many chemokines show anti-tumor activity by stimulating immune cells or by inhibiting tumor neovascularization, other chemokines may promote tumor growth and metastasis by growth stimulation, enhancing cell motility or angiogenesis. Regarding the role of chemokines in tumor metastasis, recent reports suggested that chemokine receptors CXCR4 and the like play significant roles in metastasis of melanoma, breast and ovarian cancers (Non-patent documents 1-3).

Patent document 1 discloses a method of inhibiting metastasis or maintenance of cancer cells by blocking signaling through a chemokine receptor such as CCR7, CXCR4 or CCR10. However, it is unclear whether or not expression of such chemokine receptors on cancer cells are related to metastasis of said cancer cells. Furthermore, Patent document 1 does not mention about metastasis to a lymph node which is one of the earliest metastatic foci and is an important organ in the distant metastasis.

In contrast, another chemokine receptor CXCR3 is known to be expressed on some tumors including melanoma and malignant lymphoma, and is reported to mediate chemotaxis in these cancer cells in an experiment using cultured cells (Non-patent documents 4, 5). However, its role in the metastasis of cancer and even whether or not it is responsible for metastasis are not known.

Patent document 2 discloses that stimulating function of CXCR3 receptor with CXCR3 ligand could be effective on infectious diseases and cancer, from the viewpoint that CXCR3-mediated signal transduction selectively stimulates leukocyte function which is useful in the treatment of infectious diseases and cancer. Examples of such a promoter of CXCR3 function include antibodies, homologues of natural ligand originating in other species, and substances capable of promoting receptor function without binding to the receptor. However, the role of CXCR3 in the development of cancer especially metastatic cancer has not been elucidated enough to meet the demand for the development of an effective therapeutic method for treating cancer, particularly metastatic cancer, more particularly lymph node metastasis.

When T cells are activated by IFN-γ to differentiate into Th1-type lymphocytes, CXCR3 is expressed thereon. Further, its ligands, i.e., CXCL9, CXCL10 and CXCL11, are also up-regulated by IFN-γ, attracting Th1 cells to the sites of local inflammation. It has been demonstrated that CXCL9 and CXCL10 are responsible, at least in part, for the anti-tumor effect of IL-12 that is mediated by IFN-γ (Non-patent document 6). In fact, CXCL9 gene therapy combined with an antibody-IL-2 fusion protein suppresses growth and lung metastasis of mouse colon carcinoma cells (Non-patent document 7). CXCL9 also promotes tumor necrosis when injected directly into the tumor tissue (Non-patent document 8). Notably, CXCL9 and CXCL10 act adversely on anti-tumor effect in tumor microenvironment. For example, CXCL9 activates RhoA and Rac1, induces actin reorganization, and triggers migration and invasion (Non-patent document 4).

As mentioned above, it has not been elucidated yet in what manner CXCR3 participates in the survival of cancer, further, in the metastasis thereof.

### Patent documents

- 1:: WO03/516324
- 2:: JP-A-2002-513388 (WO98/11218)

### Non-patent documents

- 1:: Muller, A. et al., Involvement of chemokine receptors in breast cancer metastasis. Nature, 410: 50-56, 2001.
- 2:: Wiley, H. E. et al., Expression of CC chemokine receptor-7 and regional lymph node metastasis of B16 murine melanoma. J. Natl. Cancer Inst., 93: 1638-1643, 2001
- 3:: Scotton, C. J. et al., Epithelial cancer cell migration: A role for chemokine receptor? Cancer Res., 61: 4961-4965, 2001
- 4:: Robledo, M. M. et al., J. Expression of functional chemokine receptors CXCR3 and CXCR4 on human melanoma cells. J. Biol. Chem., 276: 45098-45105, 2001
- 5:: Trentin, L., et al., The chemokine receptor CXCR3 is expressed on malignant B cells and mediates chemotaxis. J. Clin. Invest., 104: 115-121, 1999 6: Tannenbaum, C. S. et al., The CXC chemokines IP-10 and Mig are necessary for IL-12 -mediated regression of the mouse RENCA tumor. J. Immunol., 161: 927-932, 1998
- 7:: Ruehlmann, J. M. et al., MIG (CXCL9) chemokine gene therapy combines with antibody-cytokine fusion protein to suppress growth and dissemination of murine colon carcinoma. Cancer Res., 61: 8498-8503, 2001.
- 8:: Sgadari, C. et al., Mig, the monokine induced by interferon-γ, promotes tumor necrosis in vivo. Blood, 89: 2635-2643, 1997

### DISCLOSURE OF THE INVENTION

A purpose of the present invention is to develop a novel method for the prevention and treatment of cancer, in particular, to establish cancer therapy showing a long-term effectiveness through prevention of survival and metastasis of cancer.

The present inventors have studied intensively to elucidate the implications of chemokines-chemokine receptors in cancer survival and metastasis, and found that CXCR3, one of chemokine receptors of which involvement in cancer metastasis had not been known so far, plays a key role in the survival and metastasis of cancer cells, mainly in the metastasis to lymph nodes, and established the present invention.

Thus, the present invention relates to:
(1) A pharmaceutical composition for the treatment of cancer, characterized in that it comprises a CXCR3 inhibitor.
(2) The composition according to (1), wherein the cancer is metastatic cancer expressing CXCR3.
(3) The composition according to (1) or (2), wherein the cancer is selected from the group consisting of melanoma, breast cancer, intestine cancer and ovary cancer.
(4) The composition according to any one of (1) to (3), which is to inhibit or suppress metastasis of cancer cells to lymph node or survival of cancer cells.
(5) The composition according to any one of (1) to (4), wherein the CXCR3 inhibitor is selected from the group consisting of (A) an inhibitor of signaling through CXCR3 in cancer cells and (B) an inhibitor of CXCR3 expression.
(6) The composition according to (5), wherein (A) the inhibitor of signaling through CXCR3 in cancer cells is selected from the group consisting of a CXCR3 antagonist, an antibody against CXCR3 ligand and a fragment having the antigen binding activity thereof; and (B) the inhibitor of CXCR3 expression is selected from the group consisting of a CXCR3 antisense, a siRNA and a CXCR3 expression inhibitor.
(7) The composition according to (6), wherein the CXCR3 ligand is CXCL9, CXCL10 or CXCL11.
(8) The composition according to (6) or (7), wherein the CXCR3 antagonist includes an anti-CXCR3 antibody, a fragment having the antigen binding activity of anti-CXCR3 antibody, a mutant of CXCR3 ligand, an inhibitor of ligand binding, and an anti-idiotype antibody.
(9) The composition according to (6), wherein the CXCR3 antisense is a DNA having sequence complementary to a DNA fragment in a coding sequence or 5' non-coding sequence of DNA comprising the base sequence set forth in SEQ ID NO: 2 or an RNA corresponding to the DNA, or a chemically modified variant thereof.
(10) A method for treating cancer or preventing metastasis of cancer, which comprises administering the composition of CXCR3 inhibitor according to any one of (1) to (9) to a subject in need of such treatment.
(11) The method according to (10), wherein the cancer is metastatic cancer expressing CXCR3
(12) The method according to (10) or (11), wherein the metastatic cancer is selected from the group consisting of melanoma, breast cancer, intestine cancer and ovary cancer.
(13) The method according to any one of (10) to (12), wherein the metastasis is metastasis to lymph node.
(14) A method of screening drugs for the treatment of cancer expressing CXCR3, comprising the steps of:
   1) contacting a test compound with a cell expressing CXCR3; and
   2) determining whether the test compound inhibits CXCR3.

### BRIEF DESCRIPTION OF DRAWINGS

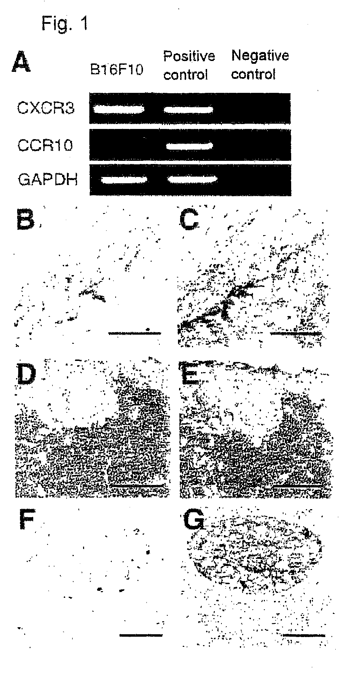

Figure 1 (A) shows the chemokine receptor expression in mouse melanoma cell line B16F10 detected by RT-PCR. (B) to (G) are copies of photographs showing the immunohistochemical staining of primary transplanted tumor and metastatic foci in the syngeneic mouse (C57BL/6) 3 weeks after inoculation

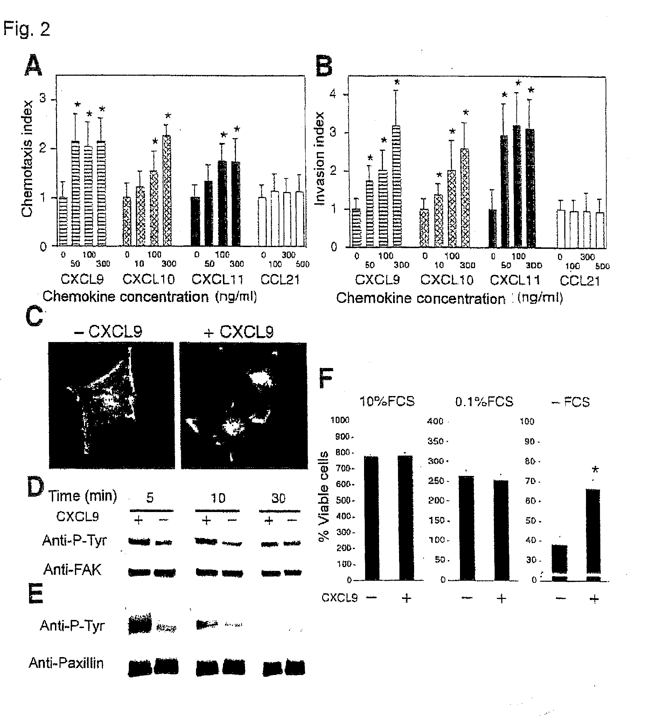

Figure 2 shows CXCR3-mediated migration, invasion, cytoskeletal rearrangement, phosphorylation of FAK and paxillin, and cell survival promotion. (A) and (B) show the chemotactic (A) and invasive (B) responses to CXCL9, CXCL10, CXCL11 and CCL21 obtained in the chemotaxis and chemoinvasion assays, respectively. (C) is a copy of photograph of fluorescence staining of actin cytoskeleton with phalloidin after incubation with or without CXCL9, showing polarized image (+CXCL9) and diffusely-stained image in the whole cell (-CXCL9). D and E are copies of photographs obtained in the Western blot analysis for examining the phosphorylation of FAK (E) and paxillin (F) after incubation with CXCL9 for the indicated time. (F) is a graph showing the number of viable cells following 48-hour-culture in a serum-containing (10% and 0.1%) or a serum-free medium after adding CXCL9 (100 ng/ml).

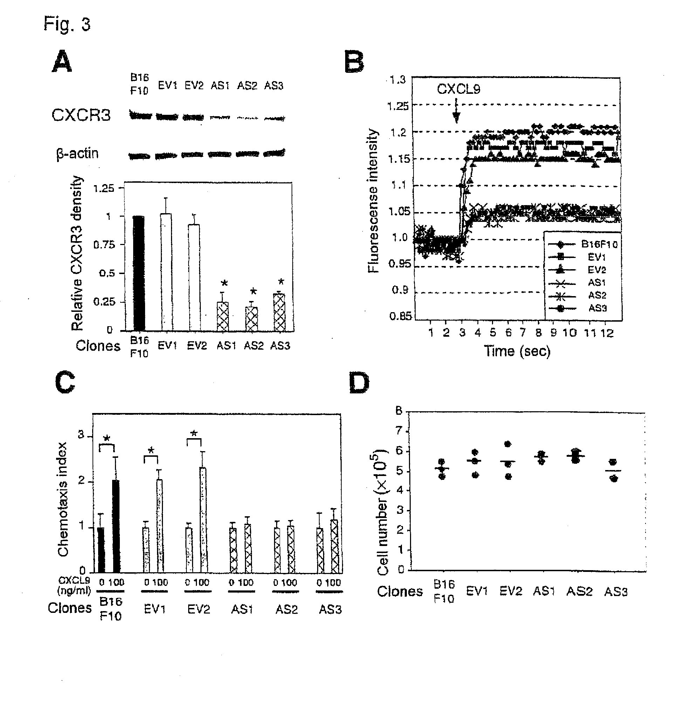

Figure 3 illustrates characterization of B16F10 cell transfected with antisense-CXCR3. (A) is a copy of Western blot photographs of CXCR3 and β-actin (top), and quantified data (bottom). (B) is a graph showing changes in the increased intracellular Ca²+ concentration upon exposure to CXCL9. (C) is a graph showing the response to CXCL9 (100 ng/ml) obtained in the chemotaxis assay. (D) shows the result of cell proliferation assay (after 48 h).

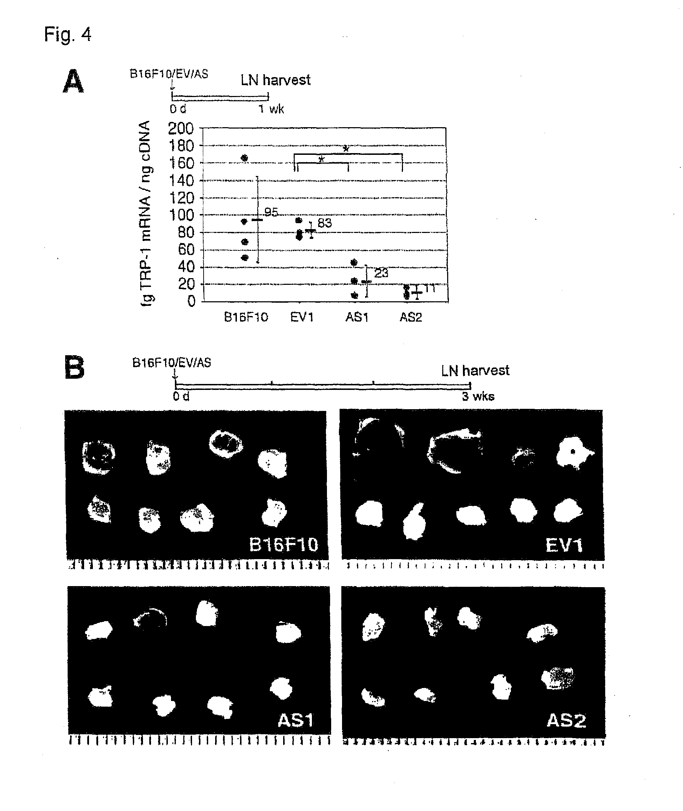

Figure 4 shows the result of analysis wherein the suppression of B16F10 metastasis to lymph node due to decrease of CXCR3 expression was examined. (A) is a graph of quantification of tumor marker TRP-1 detected in lymph node by quantitative RT-PCR. (B) shows copies of representative photographs of the popliteal lymph nodes from mice inoculated with parent, EV1, AS1 and AS2 cells (one lymph node was obtained from each mouse).

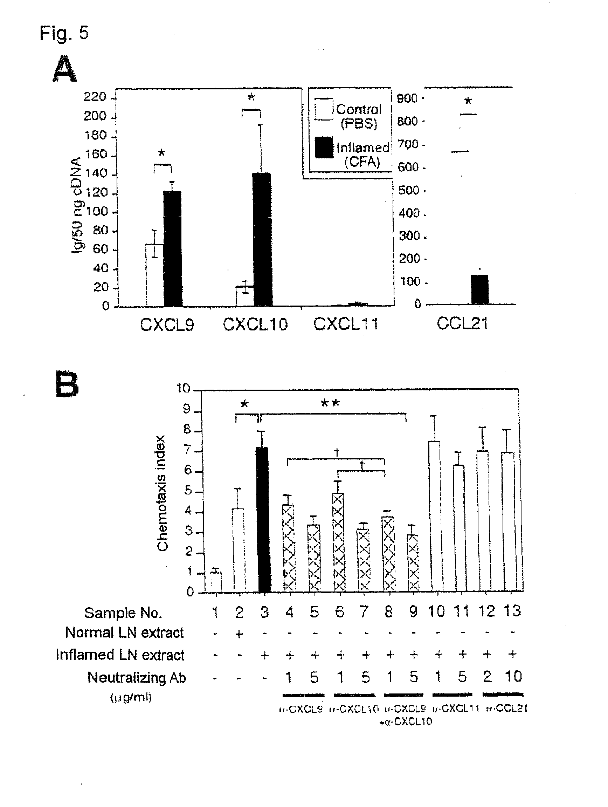

Figure 5 shows the result of analysis wherein the expression of chemokine in lymph node, and migration of cells toward the protein extracts of lymph node were examined. (A) is a graph showing quantitative RT-PCR analysis of chemokines in the inflamed lymph node 3 days after CFA or PBS injection. (B) is a graph showing the result of chemotaxis assay for the protein extract (1mg) from lymph node.

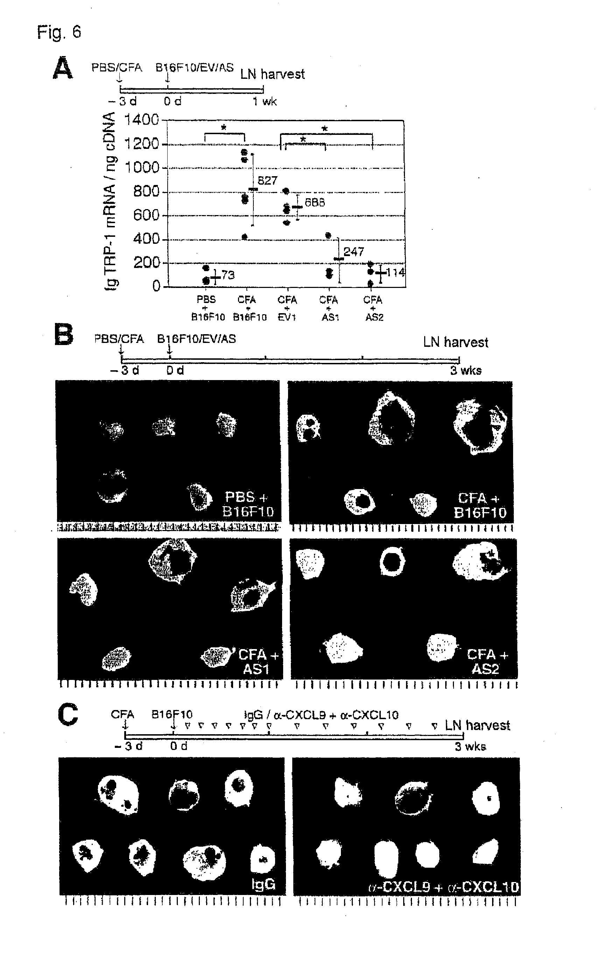

Figure 6 shows the results of analysis wherein the stimulation of B16F10 metastasis to lymph node by treatment with CFA, and the suppression of its metastasis to lymph node by inhibition of CXCR3 expression or by specific antibodies against CXCL9 and CXCL10, were examined over time. (A) is a graph showing quantification of TRP-1 detected in lymph node by quantitative RT-PCR. (B) is a copy of representative photographs of the popliteal lymph nodes from mice inoculated with PBS+parent, CFA+parent, CFA+AS1 and CFA+AS2. (C) is a copy of representative photographs of the groups inoculated with parent cell and treated with control IgG or anti-CXCL9 + anti-CXCL10 antibodies.

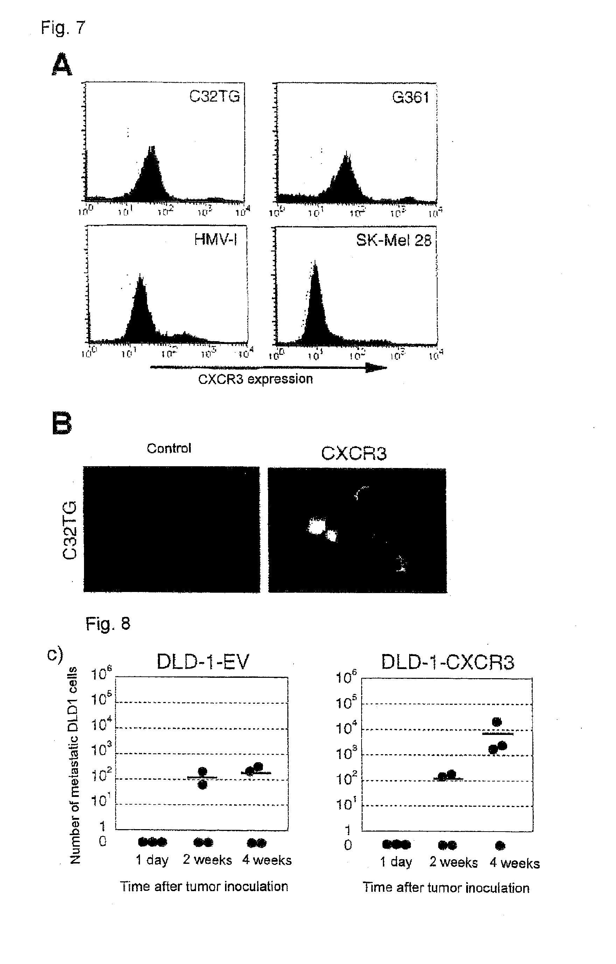

Figure 7 shows CXCR3 expression on human melanoma cell lines. (A) is the result of FACS analysis on C32TG, G361, HMV-1 and SK-Mel 28 cell lines. (B) is a copy of photographs showing the result of fluorescence staining of C32TG cells.

Figure 8 shows the number of metastatic cancer cells in periaortic lymph node counted by quantitative PCR. CXCR3-positive recombinant colon cancer cell line DLD-1-CXCR3 with forced expression of CXCR3, or CXCR3-negative DLD-1-EV was inoculated into the rectum of nude mouse, and periaortic lymph node was collected after a certain period of time from the inoculation.

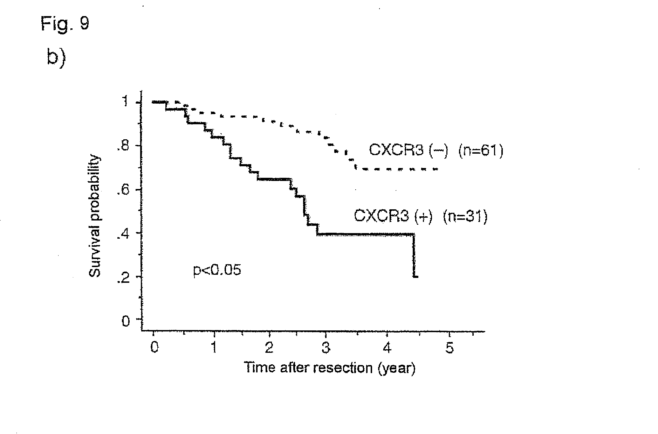

Figure 9 is a graph showing the relationship between the fact whether the colon cancer is CXCR3-positive or CXCR3-negative tumor and the survival probability after surgical resection in 92 patients of colon cancer.

### BEST MODE FOR CARRYING OUT THE INVENTION

The present invention has been established on the basis of the first finding that CXCR3 is implicated in survival and metastasis of cancer. Thus, as described in Example 1, the present inventors have found that, when B16F10 mouse melanoma cells with reduced CXCR3 expression, which have been constructed by transfection with CXCR3 antisense, were inoculated to mice (C57BL/6), metastatic frequency of cells to lymph nodes decreased; that, when the expression levels of CXCL9 and CXCL10 which are CXCR3 ligands in lymph nodes (LN) were elevated by stimulating with complete Freund's adjuvant (CFA), the metastatic frequency increased; that such increasing reaction was suppressed by anti-CXCL9 and anti-CXCL10 antibodies; and that the receptor-ligand system comprised of CXCR3 and CXCL9, CXCL10 or CXCL11 is involved in the survival and metastasis to lymph nodes of melanoma cells, and so on. Further, the present inventors revealed that the signaling through CXCR3 (CXCR3 signaling) plays an important role in lymph node metastasis of B16F10 cells, with little involvement in metastasis to lungs. Sentinel lymph nodes are the primary site where specific immune responses to the tumor antigens can be initiated, which responses affect the systemic tumor immunity. The effect of anti-tumor responses is dependent on the intrinsic immunogenicity of tumor cells and also on the modes and amounts of cancer cells migrating into sentinel lymph nodes. In fact, it is known that Lymph nodes show reactive histopathology with tumors such as lymph follicle hyperplasia, sinus histiocytosis, lymphoid cell depletion, fibrosis, angiogenesis, sarcoidlike reaction, although it remains unknown how such reactions in a host participate in cancer development. Thus, migration of cancer cells from the primary sites into sentinel lymph nodes is a critical process for both anti-tumor responses and establishment of eventual tumor metastasis. The above results demonstrate in the first place that CXCL9 and CXCL10 expressed within lymph nodes facilitate B16F10 cell metastasis by enhancing various responses of cytoskeletal rearrangement, migration, invasion, cell survival mediated by CXCR3 signal.

Further, as shown in the Example 2 below, the present inventors have found that certain human colon cancer cell lines express CXCR3 and that there is a significant correlation between CXCR3 expression and metastasis to lymph nodes. Specifically, when CXCR3-positive colon cancer cell lines with forced expression of CXCR3 were transplanted into the rectum of a mouse, the said positive cells showed higher metastatic frequency to lymph nodes than non-expressing cells. The present inventors also revealed that, in colon cancer patients having tumors expressing CXCR3 among colon cancer patients, there is a significant correlation between CXCR3 expression and lymph node metastasis and that their prognosis is poor.

The above results demonstrate that CXCR3 is expressed on a wide range of cancer cells and plays a critical role at least in metastasis to lymph nodes. Therefore, it is believed that elucidation of functions of CXCR3 or its ligands would realize treatment, prevention of metastasis and diagnosis of cancer, and the like. The present invention has been established on the basis of these findings. The present invention will be further illustrated hereinafter.

### I. Cancer

In the present claims and description, the term "cancer" has the meaning typically used in the art such as medical or pharmaceutical sciences. Generally, cancer refers to malignant tumor characterized by invasion and metastasis among tumors that involves cell proliferation with autonomy being lost. The present invention is directed to any cancer containing cancer cells expressing chemokine receptor CXCR3 on their cell surface and of which various functions for survival and metastasis are under the influence of signal transduction mediated by the said receptor CXCR3. Examples of such cancers include melanoma, and breast, intestine and ovary cancers, and the like, but not limited thereto as far as the above criterion is satisfied.

The decision (evaluation) whether or not certain cancer is the target of the present invention may be performed in principle based on whether or not cancer cells constituting said cancer are expressing CXCR3, and whether or not survival and metastasis of cancer cells depend on signal transduction mediated by CXCR3 receptor (signaling through CXCR3 or CXCR3 signaling). Screening of target cancer can be performed by utilizing a known method in the art, for example, methods which are hereinafter described in relation to assays, such as RT-PCR, immunohistochemical staining, fluorescent antibody method specifically described in Example 1 (2). A cancer cell expressing CXCR3 (a cancer cell of which survival and metastasis are under the influence of signaling through CXCR3) as described herein is also referred to as "CXCR3-positive cancer cell".

### II. Receptor CXCR3 and its ligands

In the present claims and specification, "CXCR3" means native or endogenous mammal CXCR3. Primate especially human CXCR3 is preferred. In the context of the present invention concerning CXCR3 antagonist or CXCR3 ligand, "CXCR3 (protein)" includes not only native or endogenous CXCR3 but also a protein (recombinant, synthesized protein, etc.) having the same amino acid sequence as the said native or endogenous CXCR3. CXCR3 may be isolated and purified, for example from cells, tissues or organs producing mammal CXCR3, or may be obtained from cultured recombinant host to which a known DNA sequence has been introduced. Information about base and amino acid sequences of CXCR3 can be obtained from accessible databases (NCBI, National Center for Biotechnology Information, etc.). In the database of NCBI, the base and amino acid sequences of human CXCR3 are recorded under the accession numbers of X95876 and CAA65126, respectively, and the base and amino acid sequences of mouse CXCR3 are recorded under the accession numbers of AB003174 and BAA34045, respectively, which sequences are easily available. The amino acid and base sequences of human CXCR3 are shown in SEQ ID NOS: 1 and 2, respectively.
"CXCR3 ligand" means any known chemokines for CXCR3, and examples of CXCR3 ligand of mammals (human, mouse, etc.) include CXCL9 (Mig), CXCL10 (IP-10), CXCL11. These mammalian CXCR3 ligands are preferred, human CXCR3 ligands are more preferred, and CXCL9 and CXCL10 are especially preferred. "CXCR3 ligand" refers to not only native or endogenous CXCR3 ligands but also proteins or peptides which can bind to CXCR3 and transmit specific signal, such as those (e.g., recombinant, synthesized protein, etc.) having the same amino acid sequence as the said native or endogenous CXCR3 ligand and variants maintaining the original ligand activity (e.g., variants obtained by deletion, substitution, addition of more than one amino acid).

### III. CXCR3 inhibitors (Inhibiting agents)

According to the present invention, the term "CXCR3 inhibitor" used in the claims and the specification refers to an agent which inhibits a reaction between CXCR3 and its ligands (CXCL9, CXCL10, CXCL11, etc.), which is responsible for signaling regarding survival and metastasis of CXCR3-positive cancer cells. Such inhibitors include (A) an inhibitor of signaling through CXCR3 (CXCR3 signaling) in cancer cells or (B) an inhibitor of CXCR3 expression. Examples of the above (A) include CXCR3 antagonists and antibodies against ligands for CXCR3 or fragments having antigen binding activity thereof and examples of (B) include CXCR3 antisenses, siRNAs and agents inhibiting CXCR3 expression.

In general, the term "antagonist" refers to a substance antagonizing an agonist, which is a signaling substance capable of specifically binding to a receptor and transmitting a particular information, by binding to the said receptor without transmitting such particular information. It is also called blocking agent. Accordingly, CXCR3 antagonists include CXCR3 antibodies, mutants of native ligands for CXCR3, other agents which inhibit binding of ligands, and also substances which inhibit receptor function without binding to receptors (e.g., anti-idiotype antibodies).

### (A) Inhibitors of CXCR3 signaling in cancer cells

### (i) Antibody

"Antibody" used in the present invention is a substance having an activity of inhibiting intrinsic functions of mammal CXCR3 such as binding activity, signaling activity, and the like. Such an activity enable the antibody to prevent CXCR3 from binding with a ligand and to inhibit signaling through CXCR3-ligand system selectively.

The term "ligand" of CXCR3 used hereinafter means CXCL9, CXCL10 or CXCL11, unless otherwise noted.

Antibodies against CXCR3 or any ligands may be used in the present invention as far as they show the above activity. Antibodies may be either polyclonal or monoclonal antibodies. An antibody and any peptide fragments thereof having its antigen binding activity may also be used.

Antibodies recognizing CXCR3 or its ligands and those further neutralizing their activities can be obtained by immunizing an animal appropriately with an antigen such as CXCR3 protein, ligand protein or a peptide fragment comprising an epitope thereof in a conventional manner. Commercially available antibodies are also usable.

As mentioned above, the amino acid sequence of CXCR3 and nucleotide sequence encoding the same are known, and the base and amino acid sequences of human CXCR3 are recorded in the NCBI database under the accession numbers of X95876 and CAA65126, respectively, and the base and amino acid sequences of mouse CXCR3 are recorded under the accession numbers of AB003174 and BAA34045, respectively. Based on these sequences, an antigen peptide can also be synthesized and an antibody against it (polyclonal or monoclonal antibody) may be generated. The amino acid sequence of human CXCR3 set forth in SEQ ID NO: 1 can also be used.

As described below, CXCR3 antibodies are useful in the screening for detecting cancer expressing CXCR3-positive cancer cells, immunodiagnosis, and inhibition of survival and metastasis of such cancer cells, and thereby contribute to the treatment of cancer. Immunodiagnosis may be selected as appropriate from immunoblotting, radioimmunoassay (RIA), enzyme immunoassay (ELISA), a fluorescence or luminescence assay, and the like.

As for CXCR3 ligands, CXCL9, CXCL10 and CXCL11, their amino acid sequences and base sequences encoding the same are known and are recorded in NCBI under the following accession numbers. The accession numbers of base and amino acid sequences of human CXCR9 are BC063122 and AAH63122, respectively; those of base and amino acid sequences of human CXCL10 are BC010954 and AAH10954, respectively; and those of base and amino acid sequences of human CXCL 11 are AF030514 and AAC39775, respectively. Based on the sequence information above, one can prepare antibodies (polyclonal or monoclonal antibodies) against CXCL9, CXCL10 or CXCL11.

Polyclonal and monoclonal antibodies can be prepared by a known method, for example, as described in Antibodies; A Laboratory Manual, Lane, H, D. et al. ed., Cold Spring Harber Laboratory Press publication, New York 1989, Kohler et al. eds.; Nature, 256: 495-497 (1975); Eur. J. Immunol. 6: 511-519 (1976); Milstein et al., Nature 266: 550-552 (1977); Koprowski et al., U.S. Pat. No. 4,172,124) etc. The process is illustrated below.

### (1) Polyclonal antibody

To obtain a polyclonal antibody against a specific protein or a peptide fragment thereof, an animal is immunized with the protein or the peptide as an antigen. Immunization is performed by intravenous, subcutaneous or intraperitoneal administration to a mammal (e.g., rat, mouse, rabbit, human, etc.). The immunization is conducted 1 to 10 times, preferably 4 to 5 times at intervals of several days to several weeks, preferably 2 to 3 weeks, but not limited thereto. Antibody titer is measured after 7 to 10 days from the final immunization, and antiserum is obtained from blood collected on day when the antibody titer reached maximum. Antibody titer can be measured by enzyme-linked immunoassay (ELISA), radioimmunoassay (RIA), immunohistological staining, and the like.

When necessary, antibody can be purified from antiserum by an appropriate method selected from conventional methods such as ammonium sulfate precipitation, ion exchange chromatography, gel filtration, affinity chromatography, or a combination thereof.

### (2) Monoclonal antibody

### 1) Immunization

To obtain a monoclonal antibody an animal is immunized with an antigen protein or a peptide fragment thereof. Immunization is performed by intravenous, subcutaneous or intraperitoneal administration to a mammal (e.g., rat, mouse, etc.). The dosage of antigen can be, in the case of mouse, 30 µg /animal. The immunization is conducted 4 to 5 times at least at intervals of several days to several weeks, preferably 2 to 3 weeks, but not limited thereto. After the final immunization, antibody-producing cells are collected. Splenocytes are preferred antibody-producing cells.

### 2) Cell fusion

Antibody-producing cells such as splenocytes are then fused with myeloma cells. As myeloma cells, commonly available established cell lines originated in an animal such as mouse may be used. Cell lines usable preferably are those having drug selectivity, and being capable of surviving in HAT selective medium (containing hypoxanthine, aminopterin and thymidine) only in the fused form with antibody-producing cells, but incapable of surviving there in the unfused form. Examples of myeloma cells include mouse myeloma cell lines such as P3X63-Ag, X63Ag8.653, and the like, but not limited thereto. The cell fusion is carried out by combining antibody-producing cells and the myeloma cells at a given ratio (e.g., 3:1) in a medium for animal cell cultivation, such as serum-free DMEM or RPMI-1640, in the presence of a cell fusion promoting agent such as polyethylene glycol or while treating by electrical pulse (e.g., electroporation).

### 3) Selection of hybridomas

Hybridomas are then selected by, for example, culturing cells in a medium containing hypoxanthine (100µm), aminopterin (0.4µm), and thymidine (16µM). The growing cells are selected as hybridomas. Then, screening is performed to detect whether or not an antibody of interest is present in the culture supernatant of the proliferated hybridomas. The screening of hybridomas may be carried out by any conventional methods. For example, a part of the culture supernatant of a well wherein hybridomas are grown is collected and subjected to immunostaining method, ELISA or RIA, etc. for screening.

### 4) Cloning

A fused cell can be cloned by limiting dilution method or the like to eventually establish a hybridoma as a monoclonal antibody-producing cell. Monoclonal antibodies are isolated from the established hybridoma by a conventional cell culture method or the like.

According to the cell culture method, hybridomas are cultured in an animal cell culture medium such as 10% fetal bovine serum-containing RPMI-1640 medium or MEM medium under usual culture conditions (e.g., 37° C, 5% CO₂ concentration) for 14 days, for example, and monoclonal antibodies are recovered from the culture supernatant.

### 5) Purification

If necessary, antibodies can be purified by an appropriate method selected from known methods such as ammonium sulfate precipitation, ion-exchange chromatography, affinity chromatography and gel chromatography, or a combination thereof.

The monoclonal antibody of the present invention can be selected based on mass spectrometric analysis of proteins isolated by means of electrophoreses of immunoprecipitate.

### (3) Other methods

Antibodies can also be obtained by selection of antibody libraries in phage or similar vectors (Huse, et al. (1989) "Generation of a Large Combinatorial Library of the Immunoglobulin Repertoire in Phage Lambda," Science 246:1275-1281; and Ward, et al. (1989) Nature 341:544-546).

### (4) Modification of antibodies

Antibodies may be in the form of chimeric or humanized antibodies. When applying to human, it is especially preferred to humanize antibodies to minimize immune response. Method of producing humanized antibodies are known to those skilled in the art. Further, antibodies can be appropriately labeled with a detectable label such as radionuclides, enzymes, substrates, cofactors, inhibitors, fluorescent moieties, chemiluminescent moieties and magnetic particles.

### (ii) Other agents which inhibit signaling

Besides antibodies, substances having specific binding ability can also be used provided that they specifically bind to CXCR3 or ligands therefor and inhibit CXCR3 signaling. Examples of such substances include variants of CXCR3, CXCL9 or CXCL10 variants, polymers and chemical reagents.

### (iii) Use of inhibitors of CXCR3 signaling in cancer cells

Examples of methods usable for diagnosing or treating cancers containing CXCR3-positive cancer cells utilizing "an inhibitor of CXCR3 signaling in cancer cells" such as CXCR3 antagonists (e.g., CXCR3 antibodies) or antibodies against CXCR3 ligands include the following methods, but are not limited thereto.

An inhibitor above is administered to a patient having CXCR3-positive cancer as it is or after coupling to a toxin or a radionuclide, whereby signaling through chemokine receptor CXCR3 is inhibited, cancer cells are killed and/or metastasis of cancer cells is prevented.

Also, an inhibitor such as antibody is useful for drug targeting by conjugating to a drug or other therapeutic agent, either directly or indirectly by means of a linker.

By using the above inhibitor, drug screening can be performed to identify candidate compounds having capacity to bind CXCR3, and/or to block chemoattraction to chemokine, Ca⁺⁺ flux, or the reaction of CXCR3-ligand. An appropriate biological assay can then be utilized to determine if the candidate compound has intended binding or blocking activity, e.g., an antagonist activity. Low-molecular substances are also included in screening targets. Screening methods for low-molecular substances which inhibit ligand binding to the receptor are known in the art. Such molecules can compete with a natural CXCR3 ligand, i.e., CXCL9, CXCL10 or CXCL11, and selectively bind to CXCR3 and thereby blocking the binding of natural ligand. Further, it is possible to screen drugs for the activity of blocking signaling pathways downstream from the chemokine signaling pathways.

### (iv) Immunoassays

Immunoassays are utilized in diagnosing whether or not certain cancer is responsive to the composition or method of the present invention. Immunoassays can be performed by any of known methods (e.g., Stites and Terr (eds. 1991) Basic and Clinical Immunology ,7th ed.). A suitable immunoassay allows to evaluate expression of CXCR3 on cell surface and to diagnose primary or metastatic cancers susceptible to the composition or method of the present invention, which enables efficient treatment. For example, evaluation is made on the basis of the numbers or types of cancer cells expressing CXCR3 in some cancer tissue. To treat cancer or prevent metastasis of cancer, these cancers may be labeled specifically.

Immunoassays for measurement of receptor proteins or peptides can be performed by a variety of methods known to those skilled in the art. Examples of immunoassays include competitive binding assay and noncompetitive binding assays.

In the former assay, the substance to be tested in a sample competes with a labeled analyte for specific binding sites on a capture agent being immobilized. The capture agent is preferably an antibody specifically reactive with CXCR3 as described above. The concentration of labeled analyte bound to the capture agent is inversely proportional to the amount of free analyte present in the sample.

In competitive binding immunoassay, the test substance present in the sample competes with labeled protein for binding to a immobilized or non-immobilized specific binding agent, e.g., an antibody specifically reactive with CXCR3. An appropriate technique may be used to separate the bound labeled protein from the unbound labeled protein and the amount of bound labeled protein is determined. The amount of test substance present in the sample is inversely proportional to the amount of labeled protein bound.

A homogeneous immunoassay may also be utilized in which a separation step is not required. In this immunoassay, the label on the labeled protein is altered by the binding to its specific binding agent, and this alteration results in, for example, decrease or increase in the signal emitted by label. Therefore, measurement of the label at the end of the immunoassay allows for detection or quantitation of the protein to be analyzed.

Diagnostic detection of CXCR3 may also be performed by a variety of known noncompetitive immunoassays (e.g., two-sites solid-phase sandwich immunoassay). In this assay, for example, a binding agent for the protein (e.g., an antibody) is attached to a solid support and a second protein binding agent (e.g., an antibody which binds the protein at a different site) is labeled. After binding at both sites on the protein to be analyzed has occurred, the unbound labeled binding agent is removed and the amount of labeled binding agent bound to the solid phase is measured. The amount of labeled binding agent bound is directly proportional to the amount of protein to be analyzed in the sample.

Western blot analysis can be performed to determine the presence of CXCR3 protein in a sample obtained from a patient. For example, electrophoresis is carried out on a tissue sample suspected to contain CXCR3 protein to separate the protein, and the protein is transferred to a suitable solid support (e.g., a nitrocellulose filter), then the solid support is incubated with a primary antibody reactive with the protein. The primary antibody may be labeled. Detection of CXCR3 protein in a sample is then effected by incubating with a second labeled antibody that binds to the primary antibody.

Labeling of components used in the above immunoassay may be performed directly or indirectly according to methods known in the art. Typically, a radioactive label incorporating ³H, ¹²⁵I, ³⁵S, ¹⁴C, or 32P is used. Non-radioactive labels include ligands which bind to labeled antibodies, fluorophores, chemiluminescent agents, enzymes, etc. The choice of label depends on sensitivity required, ease of labeling, stability, and available instrumentation. Various labeling or signal producing systems, which can be used, are disclosed in U.S. Pat. No. 4,391,904 and the like.

### (v) Treatment of cancer and prevention of cancer metastasis with an inhibitor of CXCR3 signaling

To prevent or suppress survival and metastasis of CXCR3-positive cancer cells using an agent which inhibit signaling through CXCR3 (hereinafter referred to as "CXCR3 inhibitor") of the present invention, an effective amount of CXCR3 inhibitor is administered independently or in combination with other drugs to a patient in need of treatment, etc. in a suitable route.

The CXCR3 inhibitor of the present invention may be administered in combination with other therapies for cancer (chemotherapy, radiation therapy, immunotherapy, or surgical method), and also alkylating agents, nucleic acids, antimetabolites, antimitotic toxin, antihormones, therapeutics for various symptoms (e.g., analgesics, diuretics, antidiuretics, antivirals, antibiotics, nutritional supplements, anemia therapeutics, blood clotting therapeutics, bone therapeutics, and psychiatric and psychological therapeutics).

The composition comprising a CXCR3 antagonist, an antibody against a ligand, etc. may be sterilely mixed with suitable pharmaceutically acceptable carriers or excipients to prepare, for example, an injectable formulation (solution, suspension, emulsion) or an implant. The composition of the present invention is typically administered parenterally, preferable intravenously. Since protein or peptide antagonists may be immunogenic to human subjects, they may be preferably administered slowly by a conventional IV administration set or from a subcutaneous depot. The antagonist such as an antibody can be also administered in aqueous vehicles such as water, saline, or buffers with or without various additives and/or excipients. Alternatively, the antagonist may be contained in a suspension, such as a zinc suspension. Such a suspension can be used for subcutaneous (SC), intradermal (ID), or intramuscular (IM) injection. The concentrations of a CXCR3 inhibitor are, but not limited to, in the range of about 5 to 30 mg/ml, preferably 10 to 20 mg/ml.

The dose of CXCR3 inhibitor is determined by a clinician based on various factors including the purpose of the treatment, the patient's weight, age, and condition. For the treatment of cancer, the dose begins with an amount somewhat less than the optimum dose and it is increased by small increments thereafter until the desired effect is achieved with monitoring side effects. For example, the determination of optimum dose is performed by obtaining a certain amount of sample from the patient and monitoring the number of cells expressing CXCR3 in the sample. Alternatively, to prevent metastasis of cancer cells, the administration is performed according to the above dosage regimen for cancer treatment. Such regimen is appropriately decided by the clinician. The dosage also may vary depending on the selected CXCR3 inhibitor and severity of the condition of the subject being treated.

The total weekly dose of antibodies or their fragments, which specifically bind to ligand, ranges generally from about 0.01 mg, more generally from about 0.1 mg, preferably from about 1mg per kilogram body weight. Although higher limit differs depending on the case, the total weekly dose is generally less than 1000 mg, preferably less than 100 mg, and more preferably less than 10 mg per kilogram body weight.

The total weekly dose of CXCR3 antagonist (e.g., antibody, binding fragment) ranges generally from about 0.01 mg, more generally from about 0.1 mg, preferably from about 1mg per kilogram body weight. Although higher limit differs depending on the case, the total weekly dose is generally less than 1000 mg, preferably less than 100 mg, and more preferably less than 10 mg per kilogram body weight.

The doses of other CXCR3 inhibitors are appropriately decided depending on the case with reference to the above regimen. The term "effective amount" means an amount of CXCR3 inhibitor which is efficient to achieve a desired effect of treatment for cancer cells. For example, it means not only an amount to cause extinction of metastasis or primary tumor but also an amount sufficient to ameliorate a symptom, size or growth of tumor. Those skilled in the art will understand that such desired effect of the treatment varies broadly according to the individual case.

### (B) Inhibitors of CXCR3 expression in cancer cells

### (i) Antisense to CXCR3

In the present specification, the term " a DNA having sequence complementary to a DNA fragment in a coding sequence or 5' non-coding sequence of DNA comprising the base sequence set forth in SEQ ID NO: 2 or an RNA corresponding to the DNA" refers to an antisense strand DNA of double-stranded DNA or RNA corresponding to the antisense strand DNA (hereinafter, referred to as "antisense oligonucleotide"), which binds to DNA or RNA thereby regulating CXCR3 expression. The antisense oligonucleotide may be, for example, prepared as a DNA based on the base sequence of CXCR3 protein coding gene, or prepared as an RNA by incorporating this DNA into an expression plasmid in the antisense direction. This antisense oligonucleotide may be a sequence complementary to the DNA fragment of any part of coding sequence or 5' non-coding sequence of the DNA comprising SEQ ID NO: 2, and preferably a sequence complementary to the transcription initiation site, translation initiation site, 5' non-translated region, exon-intron junction region, or 5' CAP region.

### SiRNA of CXCR3

Throughout the specification, "siRNA (short interfering RNA)" refers to a short double-stranded RNA containing 3' overhang on each strand which has a length of about 21 to 25 bases and is homologous to mRNA. Synthesis of siRNA can be carried out using CXCR3 cDNA as a template. The siRNA used in the present invention may have the same relevance to the base sequence of SEQ ID NO: 2 as the antisense oligonucleotide described above.

"Chemically modified variant" of the above "DNA or RNA corresponding to the DNA" (hereinafter referred to as chemically modified derivative of antisense oligonucleotide) refers to such a chemically modified variant that can enhance the transferability of a DNA or RNA into cells or their stability in cells. Examples of such chemically modified variant include phosphorothioate, phosphorodithioate, alkyl phosphotriester, alkyl phosphonate, alkyl phosphoamidate, and the like ("Antisense RNA and DNA", WILEY-LISS, 1992, P.1-50). These chemically modified variants can be prepared in accordance with the method described in the above reference. The antisense oligonucleotide or a chemically modified variant thereof can be used to control expression of the gene encoding CXCR3 protein, whereby to inhibit survival or metastasis of CXCR3-positive cancer cells. In case that an antisense oligonucleotide or chemically modified variant thereof is administered as such, the antisense oligonucleotide preferably has a length of, for example, 5-200 bases, more preferably 10-50 bases, and especially preferably 15-30 bases.

### (ii) Use of CXCR3 antisense

When an antisense oligonucleotide is incorporated into an expression plasmid, the length of this antisense oligonucleotide is preferably not more than 1000 bases, more preferably not more than 500 bases, and still more preferably not more than 150 bases. After inserting an antisense oligonucleotide into an expression plasmid, the plasmid is introduced into a target cell according to the standard method. The introduction may be performed a method using liposomes or recombinant viruses. The antisense oligonucleotide-expressing plasmid is, generally, prepared by inserting oligonucleotide into a standard expression vectors downstream from a promoter in a reversed direction so as to enable transcription of CXCR3 gene from 3' to 5' direction.

### siRNA of CXCR3

Upon introduction into cells, siRNA recognizes and cleaves the target mRNA in a sequence-specific manner, and thereby inhibiting expression of the target gene. For introduction into cells, siRNA can be used as it is or as an siRNA expression vector. ("RNAi Experiment protocol" Yodosha)

When an antisense oligonucleotide, a chemically modified variant of antisense oligonucleotide or a siRNA, in itself is administered, it may be formulated by mixing with a stabilizing agent, buffer, solvent, etc., and then, such formulation may optionally be co-administered with, for example, an antibiotic, anti-inflammatory, or anesthetic agent. The formulation thus prepared may be administered via various routes. It is preferred to be administered every day or every few days or weeks. To avoid such frequent administrations, a sustained release mini-pellet preparation may be prepared and embedded near the affected site. Alternatively, a formulation may be gradually and continuously administered to the affected site by means of, for example, an osmotic pump. The dose is typically adjusted so that the concentration at the site of action will be 0.1 nM to 10 µM.

### IV. Screening of drugs

The present invention has revealed that CXCR3 plays a pivotal role in survival and metastasis of cancers expressing CXCR3. Therefore, cells expressing the said receptor can be used for screening of drugs for the treatment of the CXCR3-positive cancer. Such screening comprises the steps of:
(1) contacting a test compound with the cell expressing CXCR3; and,
(2) determining whether a test compound inhibit the function of CXCR3.

CXCR3-positive cells may be cells derived from melanoma or colon cancer, as described in the Examples below, or recombinant cells expressing CXCR3 obtained by incorporating CXCR3 gene into a suitable expression vector and introducing the vector into a host cell. As host cells, any prokaryotic or eukaryotic known host cells capable of expressing CXCR3 can be used. Specific examples include cells naturally expressing CXCR3 such as mouse melanoma cell line (B16F10), human melanoma cell lines (32TG, G361, HMV-1), human colon cancer cell lines (Colo205, HCT116, HT29, RKO, WiDr) described in the Examples below, and cell lines with forced (artificial) expression of CXCR3 (e.g., DLD-1-CXCR3 cell), without limitation.

Construction of expression vector and transformation procedure of host cells are known to those skilled in the art. The evaluation whether or not a function(s) of CXCR3 is inhibited can be made by a method which comprises comparing the survival and proliferation state, and the like of CXCR3-positive cells in the presence and absence of a test substance (compound), and an assay, for example, described above in relation to "Use of inhibitors of CXCR3 signaling in cancer cells" or the like.

The present invention is further illustrated by the following Examples. The examples are not to be construed as limiting the scope or content of the invention in any way.

### Example 1: CXCR3 expression in mouse melanoma

The melanoma cell used in the example below was mouse melanoma cell line B16F10. The cell provides a useful model that can cause metastasis to lymph nodes in the syngeneic mouse (C57BL/6) (Whalen, G. F. et al., Ann. Surg., 215: 166-171, 1992). Unless otherwise noted, the said cell and mouse were used.

### I. Expression of chemokine receptors in a metastatic mouse melanoma cell line (B16F10)

Expression of chemokine receptors in B16F10 cells was screened by RT-PCR.
(1) Detection of chemokine receptors by RT-PCR
   Total RNA from cultured cells was extracted using ISOGEN (Nippon Gene Co., Ltd.) according to the manufacture's protocol. Two µg of each RNA sample was reverse-transcribed and subjected to PCR under the following conditions: denaturation at 94 °C for 30 s; annealing at 58 °C for 30 s and extension at 72 °C for 1 min for total 35 cycles.
   The primers for CXCR3 were as follows:
   5'-GCCGGAGCACCAGCCAAGCCAT-3' (SEQ ID NO: 3), and
   5'-AGGTGGAGCAGGAAGGTGTC-3' (SEQ ID NO: 4).

   The primers for CCR10 were as follows:
   5'-CTGGAATCTAGGAAGTACCAC-3' (SEQ ID NO: 5), and
   5'-CCAAAAAGGCATAAAGCACCG-3' (SEQ ID NO: 6).

   GAPDH was used as the internal control to normalize the sample amounts. The result is shown in Fig. 1 (A). From Fig. 1 (A), it is apparent that B16F10 cells express CXCR3 and CCR10 as chemokine receptors (GAPDH is the internal control). Expression of other chemokine receptors was not detected in B16F10 cells (data not shown). Further, no expression was found for any of the CXCR3 ligands (CXCL9, CXCL10, CXCL11) or CCR10 ligands (CCL27 or CCL28) either (data not shown).
(2) Immunohistochemical staining and immunofluorescence (fluorescent antibody method) in mice with inoculated tumors.

CXCR3 expression was studied *in vivo* at protein level in transplanted tumors by immunohistochemical staining.

### Method and result:

B16F10 cells (5×10⁵ cell/30µl PBS) were inoculated into the footpad of syngeneic female mice (C57BL/6 mouse, 6 - 8 week-old). After 3 weeks, autopsy was performed and the primary transplanted tumors, lymph nodes and lungs were removed from respective mice to detect metastasis by immunohistochemical staining (Figs. 1 B - G).

Formalin-fixed, paraffin-embedded sections were stained with anti-CXCR3 antibody (Santa Cruz Biotechnology) by the avidin-biotin immunoperoxidase method. IgG isotype was used as a negative control. As shown in Figs. 1 (B) to (G), it was confirmed that CXCR3 was expressed in the primary transplanted tumors as well as in the metastatic foci of lymph nodes and lungs. In the figures, (B) and (C) show primary transplanted tumors, (D) and (E) metastatic lymph nodes, and (F) and (G) metastatic lung nodules. Here, (B), (D) and (F) show negative controls stained with the IgG isotype and dark spots in the controls melanin pigments produced by melanoma cells. Also, (C), (E) and (G) show the results obtained by staining with anti-CXCR3 antibody (dark spots in the figure). (Scale bars=100 µm in Fig. 1).

### II. Chemokine-mediated migration and invasion (invasiveness)

### (1) Chemotaxis assay and chemoinvasion assay

In addition to CXCL9, CXCL10 and CXCL11, CCL21 has been reported to bind to CXCR3 in mice, although with a lower affinity. By the use of these recombinant chemokines (Peprotech, Inc.), *in vitro* assays (chemotaxis assay and chemoinvasion assay) were performed to determine whether or not they induce migration and invasion.

### Method and results:

Migration (chemotaxis) and invasion (chemoinvasion) were assayed in 24-well Transwell cell culture chambers (8 µm-pore membranes, Coster Corp.) (Saiki, I. et al., Cancer Res., 50: 3631-3637, 1990).

Filters were pre-coated with fibronectin (10-20 µg/ml) or with Matrigel (30 mg/ insert). After ,B16F10 cells (5×10⁴ and 2.5×10⁵ cells/ml for chemotaxis and chemoinvasion, respectively) were added to the upper chamber and incubated for 6h (for chemotaxis), or 24h (for chemoinvasion), cells attached on the lower surface of the membrane were counted in at least five different fields (×200). To the lower chambers for the test (experimental) groups was added each kind of chemokine, but not to the chamber(s) for the control group.

At least 3 experiments were performed for each set. Chemotaxis and chemoinvasion indices were defined as the ratios of migrating cell numbers in the test groups divided by those in the controls. Chemokinesis was tested in checkerboard assays and was negative for all chemokines.

The results are shown in Fig. 2. Fig 2 (A) shows chemotactic response to CXCL9, CXCL10, CXCL11 or CCL21, in which the horizontal axis (scale) represents chemokine concentration (ng/ml) and vertical axis (scale) chemotaxis index. (B) shows chemoinvasive response, in which horizontal axis represents chemokine concentration (ng/ml) and vertical axis chemoinvasion index. (Mean ± SD (Student's t-test; *P<0.01).

As apparent from the figure, chemokines CXCL9, CXCL10 and CXCL11 stimulated (promoted) chemotaxis and chemoinvasion of mouse melanoma B16F10 cells in a dose-dependent manner, however, CCL21 did not induce either response even at 500 ng/ml. The responses of B16F10 cells to these chemokines were obtained at the concentrations (50 - 100 ng/ml) similar to those for leukocytes.

### (2) CXCL9-induced rearrangements of actin cytoskeleton, focal adhesions and cell survival

It is known that binding of a chemokine to its receptor on cultured leukocytes triggers actin polymerization which is a prerequisite for cell motility and migration (Bleul, C. C. et al., J. Exp. Med., 184: 1101-1109, 1996), which then changes actin cytoskeleton and thereby bringing about cell motility. This effect was examined in the following manner.

### Method and results

### Phosphorylation of FAK (focal adhesion kinase) and paxillin

B16F10 cells (4×10⁶ cells) were incubated for 2 h without serum on collagen-coated 6 cm dishes, followed by incubation with CXCL9 (100 ng/ml) which is a ligand for CXCR3. Then, cells were lysed with 1 ml of lysis buffer (50 mM Tris, 150 mM NaCl, 1 mM EGTA, 2 mM Na₃VO₄, 50 mM NaF, 1% NP-40, 4 mM Na₄P₂O₇ and protease inhibitors, pH 7.4) and proteins were extracted. Each 0.45 ml lysate sample was immunoprecipitated with anti-FAK antibody or anti-paxillin antibody (Upstate Biotechnology, Inc.) using protein G-Sepharose. One half of the precipitate was blotted and analyzed for phosphorylated proteins using ECL phosphorylation detection kit (Amersham Pharmacia Biotech). The other half was subjected to analysis for detection of proteins using anti-FAK antibody or anti-paxillin antibody.

The results are shown in Figs. 2 (C) to (E). P-Tyr represents phosphorylated tyrosine and anti-P-Tyr represents antibody against protein of which tyrosine residue is phosphorylated. Anti-P-Tyr in Fig. 2 (D) shows the result obtained using an antibody against phosphorylated FAK and anti-P-Tyr in (E) the result obtained using an antibody against phosphorylated paxillin.
(C): To cells were added CXCL9 (100 ng/ml), and after 5 min, fluorescence staining of actin cytoskeleton was carried out with phalloidin. Representative photographs of polarized (+CXCL9) and diffusely-stained (-CXCL9) cells are shown.
(D) and (E): Phosphorylation of FAK (D) and paxillin (E) after adding CXCL9 to cells were examined for indicated time by Western blot analysis. Western blot analysis was performed by a known method.

As shown in Fig. 2 (C), the proportion of actin filament polarized cells was increased by about 2 times (from 20.2 ± 3.0% to 38.4 ± 5.6%; *P* < 0.01). The diffusely-stained cell population was reduced to about a half (from 45.7 ± 5.0% to 22.8 ± 1.9%; *P* < 0.01).

Furthermore, CXCL9 induced phosphorylation of FAK and paxillin (Fig. 2, (D) and (E)), which phosphorylation is essential for the formation of focal adhesion complex. The phosphorylation of FAK and paxillin took place shortly after addition of CXCL9, and lasted for about 10 min, but was reduced to the baseline level after 30 min. It is known that protein kinases, including FAK, and their substrates, such as paxillin, talin and tensin, accumulate at focal adhesions organized by β1-containing integrins (Craig, S. W. et al., Curr. Opin. Cell Biol., 8: 74-85, 1996). Thus, it is conceivable that CXCL9 induces a rapid and transient upregulation of β1 integrin-mediated adhesion to the extracellular matrix in the lymph nodes.

### (3) Assays for cell growth and survival

It is known that binding of CXCL12/SDF-1α (stromal cell-derived factor 1α) with CXCR4 induces cell growth and survival in addition to chemotaxis in some cell types. It was therefore examined whether CXCL9 induces such reactions in B16F10 cells. B16F10 cells (1×10⁶ cells) were incubated for 48 h in serum-containing (0.1 and 10% FCS) or serum free media with or without CXCL9 (100 ng/ml). Viable cells were counted by the trypan blue dye exclusion method. At least 3 experiments were performed for each set. The results are shown in Fig. 2 (F). The figure shows the relation between the presence or absence of CXCL9 (horizontal axis) and the percentage of viable cells (%) (vertical axis) (Mean±SD, Student's t-test; *P<0.01)

CXCL9 did not show any effects on cell proliferation under either high density (10% FCS) or low density (0.1% FCS) serum condition. Without serum, on the other hand, CXCL9 significantly enhanced cell survival compared with the untreated control (P < 0.05).

### III. Effect of transfection with CXCR3 antisense RNA

### (1) Generation of cells (transfectants) transfected with CXCR3 antisense RNA

To examine the function of CXCR3, cells in which expression of CXCR3 was suppressed were constructed by transfection with antisense RNA (B16F10 transfectants) and analyzed.

B16F10 transfectant clonal cell line was prepared in the following manner.

A 544-bp segment at the 5' end of mouse CXCR3 cDNA (NCBI accession number: AB003174) was amplified by RT-PCR and first subcloned into TA cloning site of pCRII (Invitrogen Corp.). Then it was inserted, as a BamHI/XbaI fragment, into pcDNA3.1/Hygro (Invitrogen Corp.) in the antisense orientation.

The following primers were used to generate antisense RNA.
5'-AAGCCATGTACCTTGAGGTTA-3' (SEQ ID NO: 7)
5'-CAGACAGAGACCCCATACAAGC -3' (SEQ ID NO: 8).

The orientation of the inserted part of the subcloned gene was verified by mapping with several restriction enzymes (Hind III, Bgl II) and sequencing. B16F10 cells were transfected with the gene by calcium phosphate method and stable transfectants were selected by culturing in a selection medium with hygromycin B (200 µg/ml) for 3 weeks. Thus, three lines of cells transfected with antisense RNAs (hereinafter, referred to as "antisense-transfectant clones", AS1, AS2 and AS3) and two lines of cells transfected with empty vectors (hereinafter, referred to as "empty vector-transfectant clones", EV1 and EV2) were established.

The amount of CXCR3 protein was determined by Western blotting for 40 µg of cell lysate, correcting (normalizing) with β-actin. The results are shown in Fig. 3 (A). Western blot photographs of CXCR3 and β-actin are shown in the top, and quantified data in the bottom. The figure shows relative CXCR3 density (vertical axis) of each clone (horizontal axis). (Mean ± SD, n = 3 (Student's t-test; **P <* 0.01). The Western blot analysis in Fig. 3 (A) showed that the CXCR3 protein levels in the antisense-transfectant clones were decreased to about 20-25% of those in the parental or empty vector-transfectant clones.

To evaluate the function of CXCR3, determination of intracellular Ca²⁺ concentration (mobilization), chemotaxis assay and cell proliferation analysis were performed.
1) Determination of intracellular Ca²+ concentration (mobilization) (calcium mobilization assay) was carried out by measuring the relative fluorescence after pre-incubation with 20 µM Fluo-3 AM (Molecular Probes Inc.) and addition of CXCL9 (300 ng/ml) in a Fluoroskan Ascent FL (Labsystems).. Chemotaxis assay was performed according to the method described in (3) above. Proliferation analysis was carried out by incubating cells (1×10⁵ cells) for 48 h in a medium supplemented with 5% FBS and counting cells with a hemocytometer.
   The results are shown in Figs. 3 (B) to (D). Figure 3 (B) shows the results of determination of intracellular Ca²⁺ concentration after addition of CXCL9. It can be seen that increase of intracellular Ca²⁺ after addition of CXCL9 was suppressed in the antisense-transfectant clones. Figure 3 (C) shows the results of the chemotaxis assay for CXCL9 (100ng/ml). (Mean±SD, Student's t-test; *P<0.01). Increase of intracellular Ca²⁺ concentration is an immediate reaction that takes place in response to chemokines, and addition of CXCL9 to B16F10 cells induced 15-20% increase in the intracellular Ca²⁺ concentration. Although the empty vector transfectant clones showed similar increases, the antisense transfectant clones showed suppressed increases of only about 5% (Fig. 3B). Consistent with these results, the chemotaxis assay revealed that the CXCL9-induced migration were almost eliminated in the antisense-transfectant clones (Fig. 3(C)). Figure 3 (D) shows the result of cell proliferation assay (after 48 hrs.). To rule out possible effects of CXCR3 suppression on cell proliferation, the growth rates of the parental, empty vector transfectant and antisense transfectant B16F10 clones were determined and significant difference was not found among the clones (Fig. 3(D)).
(2) Suppressed metastasis of antisense-transfectant clones to lymph nodes.
   To examine the effect of CXCR3 on lymph node metastasis, B16F10 melanoma cells were inoculated into the hind footpads of syngeneic mice. One week after inoculations, popliteal lymph nodes (LN) were obtained (harvested), homogenized and RNA was extracted using ISOGEN (Nippon Gene Co., Ltd.) according to the supplier's instruction. RNA was treated with DNaseI to eliminate possible genomic DNA contamination, and subjected to cDNA synthesis by reverse transcription. The resulting cDNA was amplified and analyzed by quantitative RT-PCR with ABI-7700 DNA Sequence Detector (Perkin-Elmer Corp) using the following primers and probes according to the supplier's instruction. The present assay for quantifying expression level of mRNA of TRP-1 that is a melanocyte-specific marker gives a sensitive detection method for lymph node micrometastasis and hence has been employed clinically to treat human melanoma (Bieligk, S. C. et al., Ann. Surg. Oncol., 6: 232-240, 1999).

### TRP-1:

5'-CCTAGCTCAGTTCTCTGGACATGA-3' (SEQ ID NO: 9)
5'-TCGCAGGCCTCTAAGATACGA-3' (SEQ ID NO: 10)
5'-Fam-CTGCCTGGGCCACAGTTCACCTCTAATT-Tamra-3' (SEQ ID NO: 11) CXCL9:
5'-AGAACTCAGCTCTGCCATGAAGT-3' (SEQ ID NO: 12)
5'-AACTCCACACTGCTCCAGGAA-3' (SEQ ID NO: 13)
5'-Fam-CGCTGTTCTTTTCCTTTTGGGCATCA-Tamra-3' (SEQ ID NO: 14) CXCL10:
5'-CCAGTGAGAATGAGGGCCATAGG-3' (SEQ ID NO: 15)
5'-CTCAACACGTGGGCAGGAT-3' (SEQ ID NO: 16)
5'-Fam-AAGCTTGAAATCATCCCTGCGAGCC-Tamra-3' (SEQ ID NO: 17) CXCL11:
5'-CAGGAAGGTCACAGCCATAGC-3' (SEQ ID NO: 18)
5'-CAAAGACAGCGCCCCTGTT-3' (SEQ ID NO: 19)
5'-Fam-CCACAGCTGCTCAAGGCTTCCTTATGTTC-Tamra-3' (SEQ ID NO: 2 0)

### CCL21:

5'-CAAAGCAGCCACCTCATGCT-3' (SEQ ID NO: 21)
5'-ATGGCCGTGCAGATGTAATG-3' (SEQ ID NO: 22)
5'-Fam-TCCACACCCTTGCCCTGCTTCAA-Tamra-3' (SEQ ID NO: 23)

The GAPDH mRNA levels were used as an internal standard for normalization. The condition of PCR was the same as that described in the above I (1).

Serial dilution studies demonstrated that the detection limit of TRP-1 (Tyrosinase Related Protein-1) signal was as few as 50 melanoma cells per 5 × 10⁶ murine lymph node cells.

As shown in Fig. 4, B16F10 metastasis to lymph node was suppressed when CXCR3 expression decreased. Figure 4 (A) shows quantification of melanoma tumor marker TRP-1 detected in lymph nodes by the quantitative RT-PCR. Mean ± SD (Student's *t*-test; **P* < 0.05). Figure 4 (B) shows representative photographs of the popliteal lymph nodes from mice after 3 weeks from inoculations with parent cells, EV1, AS 1 and AS2, respectively. One lymph node was harvested (collected) from each mouse. Scale = 1mm.

In a week, the primary tumors in the footpads became grossly visible, and metastasis was found in lymph nodes of some mice. TRP-1 mRNA expression level from the mice inoculated with the antisense-transfectant clones (AS1 and AS2) was reduced to about 1/4 - 1/8 of that with the empty vector-transfectant clone (EV1) (*P<* 0.05). On the other hand, the level from the mice inoculated with the parental cells was essentially the same as with the empty vector-transfectant clone (Fig. 4(A)). The expression levels of TRP-1 in each cultured cells were virtually the same among the parental, empty vector- and antisense-transfectant clones (8-9 pg/ng cDNA). Tumor metastasis to lymph nodes was evaluated after three weeks from inoculations, when the primary tumors in the footpads reached about 1 cm in diameter (Fig. 4 (B)). Although the parental and empty vector-transfectant clones formed metastatic foci in 40% (10 of 25) of the lymph nodes in total, the antisense-transfectant clones metastasized to only 6.3% (1 of 16) of lymph nodes in total (Table 1; *P <* 0.05 Student's *t*-test).

In more detail, only 12.5% (1 of 8) and 0% (0 of 8) of the mice inoculated with AS1- and AS2-transfectant clones, respectively, showed metastatic foci, whereas 37.5% (6 of 16) and 44.1% (4 of 9) of the mice formed foci with the parental and EV1-transfectant clones, respectively. Regarding the transplanted primary tumors, there was no significant difference in size among mice inoculated with each clone (Table 1). Lung metastasis was also examined and no significant difference was found among these clones. Protein extracts from the primary tumors were analyzed for CXCR3 expression by Western blotting, which revealed that inhibitory effect in the antisense-transfectant clones was still maintained *in vivo*. These results demonstrate that CXCR3 plays a pivotal role in B16F10 cell metastasis to the draining lymph nodes.

**Table 1 Frequency of lymph-node metastasis**

| Inoculation | Number of metastatic LN | Primary tumor volume (mm³)^{b} |
|---|---|---|
| B16F10 (n=16) | 6/16 (37.5%) | 364 ± 115 |
| EV1 (n=9) | 4/9 (44.1%) | 322 ± 77 |
| Total (n=25) | 10/25 (40%) | |
| AS1 (n=8) | 1/8 (12.5%) | 339 ± 83 |
| AS2 (n=8) | 0/8 (0 %) | 307 ± 90 |
| Total (n=16) | 1/16 (6.3%)^{a} | |

| | | |
|---|---|---|
| ^{a} *P*<0.05 compared with "total of B16F10 and EV1" (Fisher's exact test). ^{b} Primary tumor size on day 21. Values are mean ± SD. | | |

### IV. Inhibition/suppression of human CXCR3 expression with siRNA

Inhibition of human CXCR3 expression with siRNA was examined in the following manner.

Target sites on the CXCR3 DNA and siRNA used are as shown below.
1) CXCR3siRNA1
   DNA target sequence: position 442 - 462 of SEQ ID NO: 2
   5'-AAGTGGCAGGTGCCCTCTTCA-3' (SEQ ID NO: 29)
   siRNA double strand
   sense: 5'-GUGGCAGGUGCCCUCUUCAdTdT (SEQ ID NO: 30)
   antisense: 5'-UGAAGAGGGCACCUGCCACdTdT (SEQ ID NO: 31)
2) CXCR3siRNA2
   DNA target sequence: position 162 - 182 of SEQ ID NO: 2
   5'-AACGAGAGTGACTCGTGCTGT -3' (SEQ ID NO: 32)
   siRNA double strand
   sense: 5'-CGAGAGUGACUCGUGCUGUdTdT (SEQ ID NO: 33)
   antisense: 5'-ACAGCACGAGUCACUCUCGdTdT (SEQ ID NO: 34)
3) Method and results
   Colon cancer cells (Colo205 cells) were transfected directly with CXCR3 siRNA1 or CXCR3 siRNA2 using RNAiFect Transfection Reagent (QIAGEN), and inhibition of CXCR3 (target gene) expression was examined. The protein level was determined by Western blot 24 hours after gene transfection, which revealed that CXCR3 expression was suppressed significantly to 50% in cells transfected with CXCR3 siRNA1 and also in cells transfected with CXCR3 siRNA2 compared to the expression in untransfected colon cancer cells Colo205. Expression at mRNA level was then analyzed by the semi-quantitative RT-PCR, which revealed that such expression was significantly suppressed to 32% and 66% in cells transfected with CXCR3 siRNA1 and CXCR3 siRNA2, respectively, compared to that in untransfected colon cancer cells Colo205.

### V. Effect of CFA-induced CXCL9 and CXCL10

### (1) Enhanced migration of B16F10 cells by CFA-induced CXCL9 and CXCL10

CFA (complete Freund's adjuvant)-induced expression of chemokines (CXCL9 and CXCL10) in lymph nodes and chemotaxis for the protein extracts were examined.

Both CXCR3 ligands, CXCL9 and CXCL10, are expressed at high levels only within lymphoid tissues, but not in the lung, liver or brain in the steady state (Gattass, C. R. et al., J. Exp. Med., 179: 1373-1378, 1994; Amichay, D. et al., J. Immunol., 157: 4511-4520, 1996). It is known that localized inflammation induced by CFA upregulates CXCL9 and CXCL10 in the draining lymph nodes by IFN-γ produced from Th1 cells. To identify the CXCR3 ligands present in inflamed lymph nodes, the mRNA expression levels for CXCL9, CXCL10, CXCL11 and CCL21 were determined in the lymph nodes 3 days after injection of CFA (inflammation induction) or PBS (control) by quantitative RT-PCR under the same condition as described in the above (7). In the same manner as above, localization of CXCL9 and CXCL10 within lymph nodes were examined by immunohistological staining. Also in the same manner as above, chemotaxis (migration) assay was performed using protein extracts (1 mg) from normal- and CFA-induced-lymph nodes to confirm the biological activities of chemokines induced in the inflamed lymph nodes,. The results are shown in Fig. 5.

Fig. 5 (A) shows the result of quantitative RT-PCR of chemokines in the inflamed lymph node, 3 days after CFA or PBS injection. Fiure 5 (B) shows the result of chemotaxis assay (migration) for lymph node extract proteins (1mg). **P* < 0.01, ***P* < 0.01 (Sample 3 vs. 4-9), and † *P* < 0.05 (Sample 4 or 6 vs. 8).

Upon inflammation by CFA, there were 2.0- and 6.7-fold increases in the mRNA levels for CXCL9 and CXCL10 respectively. On the other hand, little CXCL11 mRNA was detected and CCL21 mRNA decreased to one-fifth level (Fig. 5 (A)). The mRNAs for CCR10 ligands CCL27 and CCL28 were not expressed in the lymph nodes (CCL27 represents a skin-specific homeostatic chemokine).

The expression of CXCL9 and CXCL10 proteins in lymph node after inflammation by CFA or PBS was investigated in the following manner. Lymph nodes were embedded with OCT compound which is an embedding agent for preparing frozen tissue (Milles Lab), frozen, sectioned at 4 um, double-stained with antibodies for either CXCL9 (R&D Systems) or CXCL10 (Santa Cruz Biotechnology, Inc.) simultaneously with anti-CD11b antibody (Pharmingen), and allowed to react with biotinylated anti-goat secondary antibody and Fluorescein Avidin DCS (Vector Laboratories Ltd), or anti-rat Alexa594 antibody (Molecular Probes) for staining.

As a result, it was confirmed that both CXCL9 and CXCL10 were expressed mainly in the subcapsular and cortical sinuses, which loci almost coincides with the locale of CD 11b (+) cells. In this experiment, it was found that expression was significantly increased after inflammation CFA compared to inflammation by BBS (data not shown).

Tumor cells are known to initially arrive in the subcapsular sinus in lymph nodes via afferent lymphatics, where primary metastatic foci are formed. Thus, the locale of CXCL9 and CXCL10 expression coincides with the initial arrest site of the tumor cells.

Chemotaxis (migration) assay was performed for protein extracts from the CFA-induced inflamed lymph nodes to evaluate the biological activities of chemokines induced in the inflamed lymph nodes (Fig. 5(B)).

Proteins from normal and inflamed lymph nodes were extracted in Tris-HCl containing a protease inhibitor (Hujanen, E. S. et al., Migration of tumor cells to organ-derived chemoattractants, Cancer Res., 45: 3517-3521, 1985).

For neutralizing studies, protein extracts were pre-incubated with anti-CXCL9, anti-CXCL11 (R&D Systems), anti-CXCL10, and anti-CCL21 (Peprotech Ltd.) antibodies, respectively.

The extracts of CFA-inflamed lymph nodes showed migratory responses about twice as much as those of the normal lymph nodes (Sample 3). As expected, anti-CXCL9 or anti-CXCL10 antibody significantly suppressed the migratory response in a dose dependent manner (Samples 4-7), but anti-CXCL11 or anti-CCL21 antibody showed no suppression (Samples 10-13). Migration was suppressed further when anti-CXCL9 and anti-CXCL10 antibodies were combined (Samples 8-9).

### (2) Enhanced metastasis to lymph nodes by CFA-induced CXCL9 and CXCL10

Enhancement of B16F10 metastasis to lymph nodes by CFA stimulation, and suppression of metastasis by inhibition of CXCR3 expression or by specific antibodies against CXCL9 and CXCL10 were examined. The results are shown in Fig. 6. Respective experimental schedules are shown. Figure 6 A: quantification of TRP-1 detected in lymph nodes by quantitative RT-PCR. Mean ± SD (Student's t-test; **P* < 0.01, ***P* < 0.01). Figure 6 (B): representative photographs of the popliteal lymph nodes from mice injected with PBS+parental cells, CFA+parental cells, CFA+AS1 cells and CFA+AS2 cells, respectively. Figure 6 (C): representative photographs of the groups from mice injected with parental cells and treated with control IgG or anti-CXCL9 + anti-CXCL10 antibodies. (Scale = 1mm). The detail will be provided below.

To confirm whether or not CXCL9 and CXCL10 within the draining lymph nodes stimulate B16F10 metastasis, host mice were treated with CFA 3 days prior to the tumor (melanoma) cell inoculations, and after certain period of time, the TRP-1 mRNA level in lymph nodes collected from mice was investigated in the same manner as above.

As a result, one week after inoculation, the lymph nodes collected from the mice inflamed with CFA contained about 11-times more TRP-1 mRNA than those from the mice inflamed with PBS (Fig. 6(A); *P <* 0.01). This increase in the TRP-1 mRNA level by CFA-inflammation was essentially the same between the mice inoculated with the parental and empty vector clones. However, the mRNA level in the lymph node was significantly decreased to about 1/3-1/6, when mice were inoculated with the AS 1 or AS2 antisense clones compared with the EV1 clone (Fig. 6(A); *P <* 0.01).

Three weeks after inoculations, the parental B16F10 cells formed foci in 91% (21 of 23) of the lymph nodes in the host mice that had been injected (stimulated) with CFA, although the melanoma cells metastasized to only 30% (6 of 20) of lymph nodes in mice that had been pre-injected with PBS (Table 2; *P <* 0.01). However, enhancement of metastasis by CFA pre-injection was suppressed significantly when the antisense clones (CXCR3 antisense-transfectant clones) were used (Fig. 6B, Table 2; *P <* 0.01). Only 46% (7/ 15) and 33% (5/15) of mice inoculated with AS1- and AS2-transfectant clones, respectively, showed metastasis. Those metastatic foci were in relatively small size. Regarding the primary tumors, there was no significant difference in size among mice inoculated with the parental, empty vector- and antisense-transfectant clones (Table 2). Lung metastasis was examined and no difference was found among each clone.

**Table 2 Frequency of lymph-node metastasis**

| Injection | Number of metastatic LN | Primary tumor volume (mm³)^{c} |
|---|---|---|
| PBS + B16F10 (n=20) | 6/20 (30%)^{a} | 364 ± 115 |
| CFA + B16F10 (n=23) | 21/23 (91%) | 322 ± 77 |
| CFA + EV1 (n=15) | 12/15 (80%)^{b} | |
| CFA + AS1 (n=15) | 7/15 (46%)^{a} | 339 ± 83 |
| CFA + AS2 (n=15) | 5/15 (33%)^{a} | 307 ± 90 |

| | | |
|---|---|---|
| ^{a} *P* < 0.01 compared with "CFA + B16F10" (Fisher's exact test). ^{b} Not significant compared with "CFA + B16F10". ^{c} Primary tumor size on day 21. Values are Mean ± SD. | | |

To further verify the roles of CXCR3 and its ligands in B16F10 metastasis to lymph nodes, it was examined whether metastasis was suppressed by antibodies against CXCL9 and CXCL10. Namely, neutralizing antibodies (anti-CXCL9 antibody + anti-CXCL10 antibody) were repeatedly injected into the popliteal region around the lymph node, and the effects were compared with the control IgG (Fig. 6(C)). Three weeks after the inoculation of parental B16F10 cells, only 3 of 7 antibody-treated mice contained metastatic foci, although all 7 IgG-treated controls had lymph node metastasis. Furthermore, only one mouse had metastatic foci larger than 1 mm among mice treated with neutralizing antibodies, although all metastatic foci from the group treated with IgG (controls) were 2-4 mm. Thus, the above results demonstrate that CXCL9 and CXCL10 induced by CFA stimulate B16F10 metastasis to lymph nodes, and inhibition of CXCR3 activation, either by reducing the CXCR3 expression or by neutralizing the ligand chemokines, can suppress/inhibit the metastatic incidence and size of the foci.

### V. CXCR3 expression on human melanoma cells

CXCR3 expressed on melanoma cells was stained with labeled anti-CXCR3 antibody (R&D Systems Inc.) and analyzed by flow cytometry in a FACScan (Becton Dickinson & Co.). Expression of CXCR3 was examined by fluorescent antibody method. The results are shown in Fig. 7.

Four kinds of human melanoma cell lines (C32TG, G361, HMV-I and SK-Mel 28 cell lines) were analyzed by FACS, which revealed that 3 cell lines of C32TG, G361 and HMV-I cell lines expressed CXCR3, whereas SK-Mel 28 cell line did not (Fig. 7(A)). Fluorescent antibody method confirmed the expression of CXCR3 on 3 cell lines, C32TG, G361 and HMV-I (Fig. 7(B)).

The above results demonstrate that CXCR3 signaling is responsible for lymph node metastasis of B16F10 cells but less responsible for metastasis to lungs. These results indicate that target organs of melanoma metastasis is under influence of chemokine receptor(s) which is expressed therein, and CXCR3 is responsible for metastasis to lymph nodes while CXCR4 for metastasis to lungs.

### Example 2 Lymph node metastasis of human colon cancer cell lines with forced expression of CXCR3

### I. CXCR3 expression on human colon cancer cell lines

Further examination into CXCR3-positive cancers was conducted. Ten kinds of human colon cancer cell lines (Colo205, HCT116, HT29, RKO, WiDr, DLD-1, SW480, LS174T, caco2 and HCT15 cell lines) were analyzed for expression of CXCR3 with human CXCR3 antibody (R&D Systems) by Western blot analysis, and CXCR3 expression was confirmed in Colo205, HCT116, HT29, RKO and WiDr cell lines (data not shown). DLD-1, non-expressing cell line, was used in the experiments below.

### II. Lymph node metastasis of cells with forced expression of CXCR3

The relation between CXCR3 and cancer metastasis was examined using human colon cancer cell line DLD-1 not expressing CXCR3. Thus, DLD-1-CXCR3 with forced expression of mouse CXCR3 and DLD-1-EV containing an empty vector without CXCR3 were prepared using DLD-1 cells, and CXCR3 expression in respective cells was analyzed by RT-PCR. (1) Preparation of cells with forced expression of CXCR3 and functional analysis of CXCR3

### 1) Gene transfection to human colon cancer cell line DLD-1 using retrovirus

Mouse CXCR3 cDNA was amplified by RT-PCR and subcloned into retrovirus vector pMX (Onishi M et al., Exp. Hematol. 24:324-329, 1996). The sequences of primers used in the RT-PCR are shown below. The condition of PCR is the same as that described in Example 1 (1) above.
5'-GAATTCAAGCCATGTACCTTGAGGTTA-3' (SEQ ID NO: 24)
5'-CTCGAGAATTACAAGCCCAGGTAGGAG-3' (SEQ ID NO: 25)
(Underline represents restriction sites of enzyme EcoRI or XhoI.)

PCR products were fragmented with restriction enzymes EcoRI and XhoI according to the standard method and sequenced, and confirmed to be identical to the published mouse CXCR3 sequence (GenBank Accession No. AB003174). CXCR3 cDNA was designed to be integrated into the upstream of the internal ribosomal entry site (IRES) enhanced green fluorescent protein (EGFP) sequence in retrovirus vector pMX. Thus, pMX-CXCR3-IRES-EGFP was prepared.

Both pMX-CXCR3-IRES-GFP and pVSV-G (BD Biosciences) which is a plasmid encoding envelop glycoprotein of vesicular stomatitis virus (VSV-G) were transfected into GP-293 packaging cells *(*Exp. Hematol. 24:324-329, 1996 (Id.)) using FuGene6 reagent (Roche Diagnostic Corp), and the cell supernatant was recovered to obtain virus solution. In the same manner, empty virus vector not containing CXCR3 gene (pMX-IRES-GFP) was used to obtain a virus solution.

Human colon cancer cells DLD-1 (cell density: 30-50%) not expressing CXCR3 were cultured in the virus supernatant containing polybrene (8µg/ml) for virus infection. The culture condition was as follows:
Culture solution: Dalbecco's Modified Eagle Medium supplemented with 10% bovine fetal serum; and temperature: 37°C.

Infection efficiency was about 10%. The cell populations were sorted by FACS using EGFP as an index to establish a cell population highly expressing CXCR3.

In the present specification, a cell transfected with pMX-CXCR3-IRES-EGFP is referred to as "DLD-1-CXCR3 cell" and a cell transfected with an empty vector pMX-IRES-EGFP is referred to as "DLD-1-EV cell".

CXCR3 expression in respective cells was studied by RT-PCR and chemotaxis assay.

RT-PCR was performed in the same manner as above, which confirmed that CXCR3 was expressed in DLD-1-CXCR3 but not in DLD-1-EV (data not shown).

Chemotaxis assay was performed using CXCL10 as a ligand according to the method described in Example 1, II., "(1) Chemotaxis assay and chemoinvasion assay". It was revealed that DLD-1-EV failed to show chemotaxis, whereas LD-1-CXCR3 showed significant chemotaxis for 100 ng/ml CXCL10 (data not shown).

From these results, it was confirmed that CXCR3 is expressed and functions in DLD-1-CXCR3 cells.

### 2) In vivo experiments of metastasis

To investigate the role of CXCR3 in lymph node metastasis, DLD-1-CXCR3 or DLD-1-EV cells were inoculated into the rectum of nude mouse (BALB/c nude (nu/nu) mice; CLEA Japan). For the model of spontaneous lymph node metastasis of rectum cancer, suspension of human colon cancer cells (DLD-1-CXCR3 cells) (1 x 10⁶ cells/50 µl PBS) was infused into the rectal mucosa of nude mice. Control animals were inoculated with DLD-1-EV cells transfected with empty vectors in the same manner. After one week, rectum cancer was confirmed and its size increased with time. Periaortic lymph nodes were collected and the number of metastatic cancer cells were counted by quantitative PCR method 14 days and 28 days after inoculations. After 6 weeks from inoculation, periaortic lymph nodes were collected and observed under fluorescence microscopy based on GFP fluorescence to evaluate the presence of metastatic foci macroscopically. Periaortic lymph nodes were found in the area from around abdominal aorta to right and left common iliac arteries (average 2-4 lymph nodes/mouse). The results are shown in Table 3.

**Table 3 Frequency of metastasis of DLD-1 cells to LN, lung and liver**

| Cell line | Primary tumor volume (mm³)^{a} | Number of metastasis (%) | | |
|---|---|---|---|---|
| | | LN (%) | lung (%) | liver (%) |
| DLD-1-CXCR3 (n=22) | 905 ± 238 | 13 (59%)^{b} | 2 (9%) | 1 (5%) |
| DLD-1-EV (n=22) | 1028 ± 334 | 3 (14%) | 4 (18%) | 1 (5%) |

| | | | | |
|---|---|---|---|---|
| a: Values are mean ± SD. b: P < 0.01 compared with DLD-1-EV cells (Fisher's exact test) | | | | |

Metastasis to periaortic lymph nodes was found in 59 % of mice inoculated with DLD-1-CXCR3 cells with forced expression of CXCR3, while it was found in only 14% of mice inoculated with DLD-1-EV. Regarding the primary tumors, there was no difference in size among mice. Metastatic frequency to lung or liver was low and there was no difference between each group. These results demonstrate that CXCR3 has a function to facilitate metastasis of CXCR3-positive cancer to lymph nodes. These results show that CXCR3 does not affect metastasis to lung or liver, which coincide with the result of Example 1.

### 3) Quantification of metastatic cancer cells (time-course of change in quantity)

Metastasis of human colon cancer cells to lymph nodes was quantified using human β-globulin related sequence (HBB; GenBank accession No. NG 000007) as an index. DNA was extracted from lymph nodes and analyzed by quantitative PCR analysis with ABI-7700 DNA Sequence Detector (Perkin-Elmer Corp.). The condition of PCR is the same as above. The following primers and probes were used.
5'-CACTGACTCTCTCTGCCTATTGGTC-3' (SEQ ID NO: 26)
5'-AGGAGTGGACAGATCCCCAAA-3' (SEQ ID NO: 27)
5'-Fam-CTACCCTTGGACCCAGAGGTTCTTTGAGTC-Tamra-3' (SEQ ID NO: 28)

The detection sensitivity of this method confirmed by dilution test was about 50 cancer cells per lymph node.

The results are provided in Fig. 8. The figure shows that there was no difference between DLD-1-CXCR3 and DLD-1-EV after 2 weeks from inoculation; however, metastasis was increased in DLD-1-CXCR3 after 4 weeks.

### 4) CXCR3 expression in clinical specimens

To study the clinical significance of chemokine receptor in colon cancer, 92 clinical specimens were examined for CXCR3 expression by immunohistological staining. Immunostaining was performed in the same manner as above. Then, correlation between the presence of CXCR3 expression and clinicopathological factors was examined. The results are shown in Table 4.

As shown in Table 4, it was revealed that CXCR3 significantly correlated with metastasis to lymph nodes (P < 0.01), TNM stage (P < 0.01), lymphatic invasion (P = 0.016) and vascular invasion (P = 0.033). Regarding lymph node metastasis, it was found in 24 cases (77.4%) among 31 CXCR3-positive cases, whereas it was found in only 14 cases (22.9%) among 61 CXCR3-negative cases. These results indicate that there is a significant correlation between CXCR3 expression and lymph node metastasis. Further, investigation into the survival provability (rate) after surgical resection revealed that patients having CXCR3-positive tumors showed significantly lower survival rate than those having CXCR3 negative tumors, indicating worse prognosis. (Fig. 9)

**Table 4. Correlation between CXCR3 expression and various clinicopathological factors in patients with colon cancer**

| Clinicopathological factor | | CXCR3 expression | | *P* |
|---|---|---|---|---|
| | | + (n=31) | - (n=61) | |
| Age | | 67.9±10.8 | 64.6±12.5 | 0.22 |
| Sex | | | | 0.72 |
| | Male | 21 | 39 | |
| | Female | 10 | 22 | |
| Histological type | | | | 0.62 |
| | Well | 6 | 15 | |
| | Moderate | 22 | 43 | |
| | Poor | 3 | 3 | |
| Depth of tumor invasion | | | | 0.18 |
| | Tis, T1, T2 | 4 | 15 | |
| | T3, T4 | 27 | 46 | |
| Lymphatic invasion | | | | 0.016 |
| | Positive | 26 | 36 | |
| | Negative | 5 | 25 | |
| Vascular invasion | | | | 0.033 |
| | Positive | 20 | 25 | |
| | Negative | 11 | 36 | |
| Metastasis to LNs | | | | < 0.01 |
| | Positive | 24 | 14 | |
| | Negative | 7 | 47 | |
| Stage | | | | < 0.01 |
| | 1 , 2, | 4 | 41 | |
| | 3,4, | 27 | 20 | |

### NDUSTRIAL APPLICABILITY

The present invention provides an effective method for the treatment of melanoma and various kinds of cancer expressing CXCR3, and a method of preventing lymph node metastasis by suppressing/inhibiting receptor CXCR3, and further contributes to the development of cancer therapy through the prevention of refractory metastatic cancers.

## Claims

1. A pharmaceutical composition for the treatment of cancer, **characterized in that** it comprises a CXCR3 inhibitor.

2. The composition according to claim 1, wherein the cancer is metastatic cancer expressing CXCR3.

3. The composition according to claim 1 or 2, wherein the cancer is selected from the group consisting of melanoma, breast cancer, intestine cancer and ovary cancer.

4. The composition according to any one of claims 1 to 3, which is to inhibit or suppress metastasis of cancer cells to lymph node or survival of cancer cells.

5. The composition according to any one of claims 1 to 4, wherein the CXCR3 inhibitor is selected from the group consisting of (A) an inhibitor of signaling through CXCR3 in cancer cells and (B) an inhibitor of CXCR3 expression.

6. The composition according to claim 5, wherein (A) the inhibitor of signaling through CXCR3 in cancer cells is selected from the group consisting of a CXCR3 antagonist, an antibody against CXCR3 ligand and a fragment having the antigen binding activity thereof; and (B) the inhibitor of CXCR3 expression is selected from the group consisting of a CXCR3 antisense, a siRNA and a CXCR3 expression inhibitor.

7. The composition according to claim 6, wherein the CXCR3 ligand is CXCL9, CXCL10 or CXCL11.

8. The composition according to claim 6 or 7, wherein the CXCR3 antagonist includes an anti-CXCR3 antibody, a fragment having the antigen binding activity of anti-CXCR3 antibody, a mutant of CXCR3 ligand, an inhibitor of ligand binding, and an anti-idiotype antibody.

9. The composition according to claim 6, wherein the CXCR3 antisense is a DNA having sequence complementary to a DNA fragment in a coding sequence or 5' non-coding sequence of DNA comprising the base sequence set forth in SEQ ID NO: 2 or an RNA corresponding to the DNA, or a chemically modified variant thereof.

10. A method for treating cancer or preventing metastasis of cancer, which comprises administering the composition of CXCR3 inhibitor according to any one of claims 1 to 9 to a subject in need of such treatment.

11. The method according to claim 10, wherein the cancer is metastatic cancer expressing CXCR3

12. The method according to claim 10 or 11, wherein the metastatic cancer is selected from the group consisting of melanoma, breast cancer, intestine cancer and ovary cancer.

13. The method according to any one of claims 10 to 12, wherein the metastasis is metastasis to lymph node.

14. A method of screening drugs for the treatment of cancer expressing CXCR3, comprising the steps of:
1) contacting a test compound with a cell expressing CXCR3; and
2) determining whether the test compound inhibits CXCR3.
